(19) Europäisches Patentamt
European Patent Office
Office européen des brevets

(11) EP 1 572 653 B1

(12) # EUROPÄISCHE PATENTSCHRIFT

(45) Veröffentlichungstag und Bekanntmachung des Hinweises auf die Patenterteilung:
**21.11.2007 Patentblatt 2007/47**

(21) Anmeldenummer: **03815065.2**

(22) Anmeldetag: **19.12.2003**

(51) Int Cl.:
*C07D 213/38* (2006.01) *C07D 213/61* (2006.01)
*C07D 213/65* (2006.01) *C07D 405/04* (2006.01)
*A61K 31/4433* (2006.01) *A61K 31/44* (2006.01)
*A61P 25/24* (2006.01) *A61P 29/00* (2006.01)

(86) Internationale Anmeldenummer:
**PCT/EP2003/014653**

(87) Internationale Veröffentlichungsnummer:
**WO 2004/063161 (29.07.2004 Gazette 2004/31)**

(54) **GESÄTTIGTE UND UNGESÄTTIGTE HETEROARYLCYCLOALKYMETHYL-AMINE ALS ANTIDEPRESSIVA**

SATURATED AND UNSATURATED HETEROARYLCYCLOALKYLMETHYL AMINES AS ANTI-DEPRESSANTS

HETEROARYLCYCLOALKYMETHYL AMINES SATUREES ET INSATUREES EN TANT QU'ANTIDEPRESSEURS

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **20.12.2002 DE 10261091**

(43) Veröffentlichungstag der Anmeldung:
**14.09.2005 Patentblatt 2005/37**

(73) Patentinhaber: **Grünenthal GmbH**
**52078 Aachen (DE)**

(72) Erfinder:
- **BLOMS-FUNKE, Petra**
  **52146 Würselen (DE)**
- **PÜTZ, Claudia**
  **52349 Düren (DE)**
- **GRAUDUMS, Ivars**
  **52222 Stolberg (DE)**
- **KLESS, Achim**
  **52074 Aachen (DE)**
- **GRIEBEL, Carsten**
  **52070 Aachen (DE)**
- **HENNIES, Hagen-Heinrich**
  **52152 Simmerath (DE)**
- **KAULARTZ, Dagmar**
  **52222 Stolberg (DE)**
- **REINARDY, Sabine**
  **52146 Würselen (DE)**
- **SAUNDERS, Derek**
  **52072 Aachen (DE)**
- **SCHIENE, Klaus**
  **40227 Düsseldorf (DE)**
- **ENGLBERGER, Werner**
  **52223 Stolberg (DE)**
- **ZIMMER, Oswald**
  **52146 Würselen (DE)**
- **Die andere Erfinder haben auf ihre Nennung verzichtet.**

(56) Entgegenhaltungen:
**EP-A- 1 010 689** **US-A- 4 021 560**
**US-A- 4 833 143**

- **RADL S ET AL: "SYNTHESIS AND ANTINOCICEPTIVE ACTIVITY OF SOME 3-CHLOROPHENYL- AND 6-CHLOROPYRIDIN-2-YL DERIVATIVES" COLLECTION OF CZECHOSLOVAK CHEMICAL COMMUNICATIONS, ACADEMIC PRESS, LONDON, GB, Bd. 64, Nr. 2, 1999, Seiten 377-388, XP009006162 ISSN: 0010-0765**
- **M.J. O'NEIL (ED): "The Merck Index, 13th edition" 2001 , MERCK RESEARCH LABORATORIES WHITEHOUSE STATION, N.J. USA; ISBN 0911910-13-1 XP002275249 in der Anmeldung erwähnt monograph 2342: citalopram**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft gesättigte und ungesättigte Heteroarylcycloalkylmethyl-Amine, Verfahren zu deren Herstellung, Arzneimittel enthaltend diese Verbindungen und die Verwendung von gesättigten und ungesättigten Heteroarylcycloalkylmethyl-Aminen zur Herstellung von Arzneimitteln sowie Verfahren zur Behandlung von Depressionen und/oder Schmerzen.

Depression ist eine Störung der Affektivität, bei der ein depressives Syndrom im Vordergrund steht, wobei depressiv mit Verstimmung verbunden bzw. traurig gestimmt heißt. Zu den depressiven Erkrankungen werden unipolare leichte Depressionen, Dysthimie, Melancholie, bipolare Depressionen schwere Depressionen mit oder ohne Wahn, mittelgradige Depressionen, (Bipolare Erkrankung I, Manie und schwere Depression; Bipolare Erkankung II, Hypomanie und schwere Depressionen; zyklothyme Persönlichkeitsstörungen, Hypomanie und milde Depressionen) gezählt. Zur Therapie von Depressionen werden solche Antidepressiva breit eingesetzt, deren antidepressive Wirkung auf einer Wiederaufnahmehemmung der Monoamine Noradrenalin (NA) oder Serotonin (5-HT) basieren (Pacher, P., Kohegyi, E., Kecskemeti, V., Furst, S., Current Medicinal Chemistry 2001, 8, 89-100). Monoaminwiederaufnahmehemmer werden auch zur Behandlung von Angststörungen verwendet (Goddard, A. W., Coplan, J.D., Gorman, J. M., Charney, D. S., In: Neurobiology of mental illness, Charney, D.S., Nestler, E.J., Bunney, B.S. (Hrsg.), Oxford Univerity Press, New York, 1999, S. 548-563). Angststörungen werden in Generalisierte Angststörungen, Panikattacken, Zwangssyndrome, Soziale Angststörungen, Einfache Phobien, Agoraphobien, Posttraumatische Belastungsstörungen (PTSD) unterteilt.

Neben der eigentlichen antidepressiven Wirkung führen Wiederaufnahmehemmer von Noradrenalin und Serotonin auch zu einer eigenständigen analgetischen Wirkung, indem absteigende Schmerzhemmbahnen auf der Ebene des Rückenmarks aktiviert werden. Monoaminwiederaufnahmehemmer werden klinisch zur Monotherapie, aber auch als Adjuvanz zur Behandlung von chronischen Schmerzen (u.a. Neuropathischer Schmerz) eingesetzt (Sindrup, in: Yaksh, T.L., et al., Anesthesia. Biological foundations. Philadelphia: Lippincott-Raven, 1997, 987-997). Es besteht nach wie vor ein dringender Bedarf zur patientengerechten Behandlung insbesondere chronischer Schmerzen. Klassische Opiate sind bei der Therapie starker bis stärkster Schmerzen gut wirksam, bei neuropathischen Schmerzen hingegen ist ihre Wirksamkeit nicht zufriedenstellend.

Die Verwendung der Monoaminwiederaufnahmehemmer ist durch Nebenwirkungen wie z.B. Akkomodationsstörungen, Serotoninsyndrom oder QT-Verlängerungen eingeschränkt.

Eine der Erfindung zugrundeliegende Aufgabe bestand darin, neue Verbindungen zur Verfügung zu stellen, die sich zur Therapie von Depressionen, Angststörungen oder Schmerzen eignen. Darüber hinaus sollten diese Verbindungen möglichst wenige Nebenwirkungen der Monoaminwiederaufnahmehemmer wie z.B. Akkomodationsstörungen, Serotoninsyndrom oder QT-Verlängerungen aufweisen. Weitere Aufgaben bestanden darin, neue Wirkstoffe zur Behandlung von Angstzuständen, Harninkontinenz, Fibromyalgia, Essstörungen, Bulimie, Hyperaktivität, Drogenabhängigkeit, -sucht und -entzug, Trichotillomanie, Tourette's Syndrom, Hauterkrankungen wie Postherpetische Neuralgie und Pruritus, Psychosen, Gedächtnisstörungen, kognitiven Störungen und Morbus Alzheimer zur Verfügung zu stellen.

Es wurde nun gefunden, dass gesättigte und ungesättigte Heteroarylcycloalkyl-methylamine der nachstehenden allgemeinen Formel **I** die Wiederaufnahme von Noradrenalin und/oder Serotonin hemmen. Die Substanzen verfügen über ausgeprägte antidepressive, anxiolytische und antinociceptive Wirkungen und sind somit zur Behandlung von Depressionen, Angststörungen und Schmerzen geeignet. Die erfindungsgemäßen Verbindungen besitzen insbesondere ein Potential zur Therapie von chronischen Schmerzzuständen, die mit depressiven Verstimmungen oder Angststörungen vergesellschaftet sind. Darüber hinaus eignen sich die Substanzen zur Behandlung von Migräne, Harninkontinenz, Fibromyalgia, Essstörungen, Bulimie, Hyperaktivität, Drogenabhängigkeit, -sucht und -entzug, Trichotillomanie, Tourette's Syndrom, Hauterkrankungen wie Postherpetische Neuralgie und Pruritus, Psychosen, Gedächtnisstörungen, kognitiven Störungen und Morbus Alzheimer.

Aus der Literatur sind bereits 2-((Dimethylamino)methyl)-1-(pyridinyl)cyclohexanol-Verbindungen bekannt, die sich zur Behandlung von Schmerz eignen (Radl et al., Collection of Czechoslovak Chemical Communications, Academic Press, London, Bd. 64, Nr. 2, 1999, Seiten 377-388). Diese Verbindungen unterscheiden sich strukturell von den erfindungsgemäßen Heteroarylcycloalkylmethyl-Aminen durch den Cyclohexanol-Grundkörper.

Gegenstand der vorliegenden Erfindung sind daher gesättigte und ungesättigte Heteroarylcycloalkylmethyl-Amine der folgenden allgemeinen Formel **I**, auch in Form ihrer Razemate, Enantiomere, Diastereomere, insbesondere Mischungen ihrer Enantiomere oder Diastereomere oder eines einzelnen Enantiomers oder Diastereomers sowie ihrer freien Basen oder eines mit einer physiologisch verträglichen Säure gebildeten Salzes:

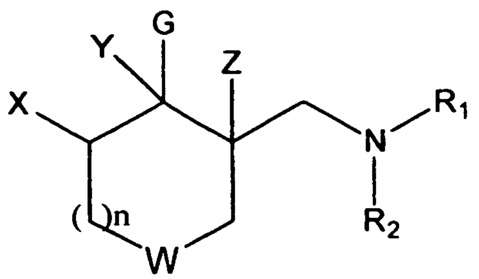

,

worin

W gleich $CH_2$ ist, wobei Y ausgewählt ist aus H oder Cl und gleichzeitig X und Z gleich H sind
oder
X und Y bilden zusammen eine Bindung und Z ist gleich H oder
Y und Z bilden zusammen eine Bindung und X ist gleich H,

oder

W gleich O, S, SO oder $SO_2$ ist, wobei Y ausgewählt ist aus H, OH, Cl und gleichzeitig X und Z gleich H sind
oder
X und Y bilden zusammen eine Bindung und Z ist gleich H
oder
Y und Z bilden zusammen eine Bindung und X ist gleich H,

und n = 0 - 3 ist.

$R^1$ und $R^2$ unabhängig voneinander ausgewählt sind aus H, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert; $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bzw. einem entsprechenden Heterocyclus, bei dem ein C-Atom im Ring durch S, O oder $NR^3$ ersetzt ist,
mit $R^3$ ausgewählt aus
H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert;
Alkylaryl, gesättigt oder ungesättigt, einfach oder mehrfach substituiert durch F, Cl, Br, I, $NH_2$, SH oder OH, $OCH_3$ oder unsubstituiert; Alkylheteroaryl, gesättigt oder ungesättigt, einfach oder mehrfach substituiert durch F, Cl, Br, I, $NH_2$, SH oder OH, $OCH_3$ oder unsubstituiert, Aryl, einfach oder mehrfach substituiert durch F, Cl, Br, I, $NH_2$, SH oder OH, $OCH_3$ oder unsubstituiert;

oder

$R^1$ und $R^2$ zusammen ein $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bilden, bzw. einen entsprechenden Heterocyclus, bei dem ein C-Atom im Ring durch S, O oder $NR^4$ ersetzt ist,
mit $R^4$ ausgewählt aus
H, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert,

und
G ausgewählt ist aus Pyridin-3-yl oder Pyridin-4-yl. G kann dabei unsubstituiert oder einfach oder mehrfach substituiert sein.

Dabei versteht man unter Bezug auf den Rest G unter dem Begriff "substituiert" im Sinne dieser Erfindung die Substitution mindestens eines Wasserstoffrestes durch F, Cl, Br, I, CN, $CF_3$, $NO_2$, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder

OH, $OCH_3$ oder unsubstituiert; $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bzw. einem entsprechenden Heterocyclus, bei dem ein C-Atom im Ring durch S. O oder $NR^5$ ersetzt ist, mit $R^5$ ausgewählt aus
H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert;
$OR^6$, $OC(O)R^6$, $OC(S)R^6$, $C(O)R^6$, $C(O)OR^6$, $C(S)R^6$, $C(S)OR^6$, $SR^6$, $S(O)R^6$ bzw. $S(O_2)R^6$, wobei $R^6$ ausgewählt ist aus H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert; $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bzw. einem entsprechenden Heterocyclus, bei dem ein C-Atom im Ring durch S, O oder $NR^7$ ersetzt ist, mit $R^7$ ausgewählt aus
H, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert;
Alkylaryl, gesättigt oder ungesättigt, einfach oder mehrfach substituiert durch F, Cl, Br, I, $NH_2$, SH oder OH, $OCH_3$ oder unsubstituiert; Aryl oder Heteroaryl, jeweils einfach oder mehrfach substituiert durch F, Cl, Br, I, $NH_2$, SH oder OH, $OCH_3$ oder unsubstituiert;

$NR^8R^9$ $C(O)NF^8R^9$ oder $S(O_2)NR^8R^9$, wobei $R^8$ und $R^9$ unabhängig voneinander ausgewählt sind aus
H, $C_1$-$C_{18}$-Alkyl, $C_2$-$C_{18}$-Alkenyl oder $C_3$-$C_{18}$-Alkinyl, jeweils verzweigt oder unverzweigt, einfach oder mehrfach substituiert durch F, Cl, Br, I, $NH_2$, SH oder OH, $OCH_3$ oder unsubstituiert; $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bzw. einem entsprechenden Heterocyclus, bei dem ein C-Atom im Ring durch S, O oder $NR^{10}$ ersetzt ist, mit $R^{10}$ ausgewählt aus
H, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert;
Alkylaryl, gesättigt oder ungesättigt, einfach oder mehrfach substituiert durch F, Cl, Br, I, $NH_2$, SH oder OH, $OCH_3$ oder unsubstituiert; Aryl oder Neteroaryl, jeweils einfach oder mehrfach substituiert durch F, Cl, Br, I, $NH_2$, SH oder OH, $OCH_3$ oder unsubstituiert;
oder
$R^8$ und $R^9$ zusammen ein $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bilden, bzw. einen entsprechenden Heterocyclus, bei dem ein C-Atom im Ring durch S, O oder $NR^{10}$ ersetzt ist, mit $R^{10}$ ausgewählt aus
H, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert;
Alkylaryl, Aryl oder Heteroaryl, jeweils einfach oder mehrfach substituiert durch F, Cl, Br, I, $NH_2$, SH oder OH, $OCH_3$ oder unsubstituiert.

Bevorzugt sind Verbindungen, in denen W gleich $CH_2$, O oder S und n = 0 - 3 ist und $R_1$ und $R_2$ unabhängig voneinander ausgewählt sind aus H, $C_1$-$C_{10}$-Alkyl, Alkylaryl, gesättigt oder ungesättigt, einfach oder mehrfach substituiert durch F, Cl, Br, I, $NH_2$, SH oder OH, $OCH_3$ oder unsubstituiert; Alkylheteroaryl, gesättigt oder ungesättigt, einfach oder mehrfach substituiert durch F, Cl, Br, I, $NH_2$, SH oder OH, $OCH_3$ oder unsubstituiert,
oder
$R_1$ und $R_2$ zusammen ein $C_3$-$C_7$-Cycloalkyl, gesättigt oder ungesättigt, einfach oder mehrfach substituiert durch F, Cl, Br, I, $NH_2$. SH oder OH, $OCH_3$ oder unsubstituiert, bilden,
und der Rest G unsubstituiert oder durch Cl, Br oder $OR_6$, wobei $R_6$ gleich H oder $C_1$-$C_{10}$-Alkyl ist, substituiert ist

Unter dem Begriff "substituiert", der sich nicht auf die oben gegebenen Definitionen für den Rest G bezieht, versteht man im Sinne dieser Erfindung die Substitution eines Wasserstoffrestes durch F, Cl, Br, I, $NH_2$, SH oder OH, $OCH_3$, wobei unter mehrfach substituierten Resten Reste zu verstehen sind, die sowohl an verschiedenen als auch an gleichen Atomen mehrfach substituiert sind, beispielsweise dreifach am gleichen C-Atom wie im Falle von $CF_3$ oder an verschiedenen Stellen wie im Falle von $-CH(OH)-CH=CH-CHCl_2$.

Der Ausdruck "$C_1$-$C_{10}$-Alkyl" bedeutet im Sinne dieser Erfindung Kohlenwasserstoffe mit 1 bis 10 Kohlenstoffatomen. Beispielhaft seien Methyl, Ethyl, Propyl, Isopropyl, n-Butan, sek. Butyl, tert. Butyl, n-Pentan, Neopentyl, n-Butan, sek. Butyl, tert. Butyl, n-Hexan, n-Heptan, n-Octan, n-Nonan, n-Decan unsubstituiert oder ein oder mehrfach substituiert genannt.

Der Ausdruck "$C_2$-$C_{10}$-Alkenyl" bzw. "$C_2$-$C_{10}$-Alkinyl" bedeutet im Sinne dieser Erfindung Kohlenwasserstoffe mit 2 bis 10 Kohlenstoffatomen. Beispielhaft genannt seien Ethenyl, Propenyl, Butenyl, Pentenyl, Hexenyl, Heptenyl, Octenyl unsubstituiert oder ein oder mehrfach substituiert bzw. Ethinyl, Propinyl, Butinyl, Pentinyl, Hexinyl, Heptinyl, Octinyl unsubstituiert oder ein oder mehrfach substituiert.

Der Ausdruck $C_3$-$C_7$-Cycloalkyl bedeutet im Sinne dieser Erfindung cyclische Kohlenwasserstoffe mit 3 bis 7 Kohlenstoffatomen. Beispielhaft seien Cyclopropyl, Cyclobutyl, Cyclopentyl, Cyclohexyl, Cycloheptyl, Cyclohexenyl oder Cycloheptenyl gesättigt oder ungesättigt, unsubstituiert oder ein oder mehrfach substituiert genannt. Dabei versteht man im Sinne der Erfindung unter einem "entsprechenden Heterocyclus" ein $C_3$-$C_7$-Cycloalkyl, bei dem mindestens ein C-Atom im Ring durch S, O oder N ersetzt Ist Beispielhaft seien hierfür Pyrrolidin, Pyran, Thiolan, Piperidin oder Tetrahydrofuran aufgeführt.

Der Ausdruck "Aryl" bedeutet im Sinne dieser Erfindung Phenyle oder Naphthyle.

Der Ausdruck "Alkylaryl" bedeutet im Sinne dieser Erfindung mit $C_1$-$C_{10}$-Alkylen substituierte Aryle, wobei die Ausdrücke Aryl und Alkyl die gleiche Bedeutung haben wie oben.

Der Ausdruck "Heteroaryl" bedeutet im Sinne dieser Erfindung 5- oder 6-gliedrige, gegebenenfalls mit einem ankondensierten Arylsystem versehene, aromatische Verbindungen, die ein oder zwei Heteroatome aus der Gruppe von Stickstoff, Sauerstoff und/oder Schwefel enthalten. In dieser Gruppe seien beispielhaft Furan, Thiophen, Pyrrol, Pyridin, Pyrimidin, Chinolin, Isochinolin, Phtialazin oder Chinazolin aufgeführt.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind.

Besonders bevorzugt sind die folgenden erfindungsgemäßen Verbindungen sowie deren Salze:

Dimethyl-(2-pyridin-3-yl-cyclohex-1-enylmethyl)-amin und das entsprechende Dihydrochlorid **(1)**

Methyl-(2-pyridin-3-yl-cyclohex-1-enylmethyl)-amin und das entsprechende Hydrochlorid **(2)**

Dimethyl-(2-pyridin-3-yl-cyclohex-2-enylmethyl)amin und das entsprechende Hydrochlorid **(4)**

Dlmethyl-(2-pyridin-3-yl-cyclohexylmethyl)-amin und das entsprechende Hydrochlorid; Diasteroisomer 1 **(6)**

Dimethyl-(2-pyridin-3-yl-cyclohexylmethyl)-amine und das entsprechende Hydrochlorid; Diastereoisomer 2 **(7)**

Benzyl-methyl-(2-pyridin-3-yl-cyclohex-1-enylmethyl)-amin und das entsprechende Hydrochlorid **(10)**

Benzyl-methyl-(2-pyridin-3-yl-cyclohex-2-enylmethyl)-amin und das entsprechende Hydrochlorid **(11)**

[2-Chlor-2-(6-chlor-pyridin-3-yl)-cycloheptylmethyl]-dimethyl-amin und das entsprechende Hydrochlorid **(13)**

(2-Chloro-2-pyridin-3-yl-cyclohexylmethyl)-dimethyl-amin und das entsprechende Hydrochlorid **(14)**

[2-Chlor-2-(6-chlor-pyridin-3-yl)-cyclohexylmethyl]-dimethylamin und das entsprechende Hydrochlorid **(15)**

Gemisch [2-(4-Brom-6-chlor-pyridin-3-yl)-cyclohex-1-enylmethyl]-methyl-amin und [2-(4-Brom-6-chlor-pyridin-3-yl)-cyclohex-2-enylmethyl]-methyl-amin und die entsprechenden Hydrochloride **(20)**

Dimethyl-(2-pyridin-3-yl-cyclohept-1-enylmethyl)-amin und das entsprechende Hydrochlorid **(23)**

Dimethyl-(2-pyridin-3-yl-cyclohept-2-enylmethyl)-amin und das entsprechende Hydrochlorid **(24)**

Gemisch aus [2-(6-Chlor-pyrtdin-3-yl)-cyclooct-1-enylmethyl]-dimethyl-amin Hydrochlorid und [2-(6-Chlor-pyridin-3-yl)-cyclooct-2-enylmethyl]-dimethylamin Hydrochlorid **(25)**

Dimethyl-(2-pyridin-3-yl-cycloheptylmethyl)-amin und das entsprechende Hydrochlorid; Diastereoisomer 1 **(26)**

Dimethyl-(2-pyridin-3-yl-cycloheptylmethyl)-amin und das entsprechende Hydrochlorid; Diastereoisomer 2 **(27)**

Dimethyl-(2-pyridin-3-yl-cyclopent-1-enylmethyl)-amin und das entsprechende Hydrochlorid **(28)**

Dimethyl-(2-pyridin-3-yl-cyclopent-2-enylmethyl)-amin und das entsprechende Hydrochlorid **(29)**

Dimethyl-(2-pyridin-3-yl-cyclopentylmethyl)-amin und das entsprechende Hydrochlorid **(31)**

Dimethyl-(2-pyridin-3-yl-cyclooct-1-enylmethyl)-amin und das entsprechende Hydrochlorid **(32)**

Dimethyl-(2-pyridin-3-yl-cyclooct-2-enylmethyl)-amin und das entsprechende Hydrochlorid **(33)**

Dimethyl-(2-pycidin-3-yl-cyclooctylmethyl)-amin und das entsprechende Hydrochlorid **(35)**

(2-Chlor-2-pyridin-4-yl-cycloheptylmethyl)-dimethyl-amin und das entsprechende Hydrochlorid **(36)**

Dimethyl-(2-pyridin-4-yl-cyclohept-2-enylmethyl)-amin und das entsprechende Hydrochlorid **(37)**

Dimethyl-(2-pyridin-4-yl-cyclohept-1-enylmethyl)-amin und das entsprechende Hydrochlorid **(38)**

Dimethyl-(2-pyridln-4-yl-cyclohex-1-enylmethyl)-amin und das entsprechende Hydrochlorid **(39)**

Gemisch aus Dimethyl-(2-pyrldin-4-yl-cyclohex-1-enylmethyl)-amln Hydrochlorid und Dimethyl-(2-pyridin-4-yl-cyclohex-2-enylmethyl)-amin Hydrochlorid **(40)**

Dimethyl-(2-pyridin-4-yl-cyclooct-1-enylmethyl)-amin und das entsprechende Hydrochlorid **(42)**

Dimethyl-(2-pyridin-4-yl-cyclopent-2-enylmethyl)-amin und das entsprechende Hydrochlorid **(43)**

Dimethyl-(2-pyridin-4-yl-cyclopent-1-anylmethyl)-arnine und das entsprechende Hydrochlorid **(47)**

[2-(6-Chlor-pyridin-3-yl)-cyclohept-2-enylmethyl]-dimethyl-amln und das entsprechende Hydrochlorid **(48)**

[2-(6-Chlor-pyridin-3-yl)-cyclohex-1-enylmethyl]-dlmethyl-amin und das entsprechende Hydrochlorid **(49)**

3-Dlmethytamlnomethyl-4-pyridin-3-yl-tetrahydro-thlopyran-4-ol und das entsprechende Hydrochlorid; Diastereoisomer 1 **(50)**

3-Dimethylaminomethyl-4-pyridin-3-yl-tetrahydro-thiopyran-4-ol und das entsprechende Hydrochlorid; Diastereoisomer 2 **(51)**

3-Dimethylaminomethyl-4-pyridin-3-yl-tetrahydro-pyran-4-ol und das entsprechende Hydrochlorid **(52)**

Dimethyl-(4-pyridin-3-yl-5,6-dihydro-2H-pyran-3-yl-methyl)-amin und das entsprechende Hydrochlorid **(53)**

[2-(5-Methoxy-pyrldin-3-yl)-cyclohex-1-enylmethyl]-dimethyl-amin und das entsprechende Hydrochlorid **(54)**

[2-(5-Methoxy-pyridin-3-yl)-cyclohex-2-enylmethyl]-dimethyl-amin und das entsprechende Hydrochlorid **(55)**

Gemisch aus [2-(5-Methoxy-pyridin-3-yl)-cyclohept-1-enylmethyl]-dimethylamin Hydrochlorid und [2-(5-Methoxy-pyndin-3-yl)-cyclohept-2-enytmethyl]-dimethyl-amin Hydrochlorid **(58)**

Gemisch aus 5-(2-Dimethylaminomethyl-cyclohept-1-enyl)-pyridin-3-ol; Hydrochlorid und 5-(7-Dimethylaminomethyl-cyclohept-1-enyl)-pyridin-3-ol; Hydrochlorid **(59)**

[2-(5-Methoxy-pyridin-3-yl)-cycloheptylmethyl]-dimethyl-amin und das entsprechende Hydrochlorid **(60)**

[2-(5-Methoxy-pyridin-3-yl)-cyclohexytmethyl]-dimethyl-amin und das entsprechende Hydrochlorid **(62)**

Gemisch aus 2-(5-Methoxy-pyridin-3-yl)-cyclohexylmethyl]-dimethyl-amin (Diastereoisomer 1) und [2-(5-Methoxy-pyridin-3-yl)-cyclohexylmethyl]-dimethyl-amin (Diastereoisomer 2) und den entsprechenden Hydrochloriden **(63)**

5-(2-Dimethylaminomethyl-cyclohex-1-enyl)-pyridin-3-ol und das entsprechende Hydrochlorid **(64)**

Dipropyl-(2-pyridin-3-yl-cyclohex-1-enylmethyl)-amin und das entsprechende Hydrochlorid **(65)**

Dipropyl-(2-pyridin-3-yl-cyclohex-2-enylmethyl)-amin und das entsprechende Hydrochlorid **(66)**

[2-(5-Methoxy-pyridin-3-yl)-cyclooct-1-enylmethyl]-dimethyl-amin Hydrochlorid und [2-(5-Methoxy-pyridin-3-yl)-cyclooct-2-enylmethyl]-dimethylamin und die entsprechenden Hydrochloride **(67)**

[2-(6-Chlor-pyridin-3-yl)-cyclohex-1-enylmethyl]-dimethyl-amin und das entsprechende Hydrochlorid **(68)**

[2-(6-Chlor-pyridin-3-yl)-cyclohex-2-enylmethyl]-dimethyl-amin und das entsprechende Hydrochlorid **(69)**

[2-(5-Methoxy-pyridin-3-yl)-cyclooctylmethyl]-dimethyl-amin und das entsprechende Hydrochlorid; Diastereoisomer 1 **(70)**

[2-(6-Chlor-pyridin-3-yl)-cyclooct-2-enylmethyl]-dimethyl-amin und das entsprechende Hydrochlorid **(71)**

[2-(5-Methoxy-pyridin-3-yl)-cyclooctylmethyl]-dimethyl-amin und das entsprechende Hydrochlorid; Diastereoisomer 2 **(72)**

[2-(5-Methoxy-pyridin-3-yl)-cyclooct-2-enylmethyl]-dimethyl-amin und das entsprechende Hydrochlorid **(73)**

[2-(5-Methoxy-pyridin-3-yl)-cyclooct-1-enylmethyl]-dirnethyl-amin und das entsprechende Hydrochlorid **(74)**

5-(7-Dimathylarnlnomethyl-cyclohept-1-enyl)-pyridin-3-ol und das entsprechende Hydrochlorid **(75)**

5-(2-Dimethylaminomethyl-cyclohept-1-enyl)-pyridin-3-ol und das entsprechende Hydrochlorid **(76)**

5-(2-Dimethylaminomethyl-cycloheptyl)-pyridin-3-ol und das entsprechende Hydrochlorid **(77)**

Methyl-(2-pyridin-3-yl-cyclohex-2-enylmethyl)-amin und das entsprechende Hydrochlorid **(79)**.

Ein weiterer Gegenstand der vorliegenden Erfindung ist ein Verfahren zur Herstellung von gesättigten und ungesättigten Heteroarylcycloalkylmethyl-Aminen der oben angegebenen allgemeinen Formel **I**. A hat in der nachfolgenden allgemeinen Formel **II** die Bedeutung $CH_2$, O. S, SO oder $SO_2$.
Heteroarylcycloalkylmethylamine der allgemeinen Formel I werden hergestellt, indem man zunächst Cycloalkanone der allgemeinen Formel **II** mit Immoniumsalzen der Formel **III** bzw. mit Paraformaldehyd und einem Amin der Formel **IV** umsetzt. $R^{10}$ hat eine $R^1$ analoge Bedeutung, $R^{11}$ eine $R^2$ analoge Bedeutung.
Anschließend setzt man die so erhaltenen Mannich-Basen mit einer metallorganischen Verbindung der Formel **V** um, in der Z Lithium bedeutet und V eine G analoge Bedeutung hat. Die Umsetzung der Mannich-Basen mit einer lithiumorganischen Verbindung der Formel **V**, in der Z Li und V eine G entsprechende Bedeutung hat, kann in einem aliphatischen Ether beispielsweise Diethylether und/oder Tetrahydrofuran bei Temperaturen zwischen -70°C und 60°C durchgeführt werden. Im Falle, dass entweder $R^{10}$ oder $R^{11}$ oder beide gleichzeitig gleich Wasserstoff sind, setzt man in die Mannich-Reaktion Verbindungen der allgemeinen Formel **III** bzw. **IV** ein, In denen $R^{10}$ oder $R^{11}$ oder $R^{10}$ und $R^{11}$ einen Benzylrest darstellen. Dieser wird an geeigneter Stelle der Reaktionssequenz durch Umsetzung der entsprechenden Verbindungen mit katalytisch aktiviertem Wasserstoff, wobei Platin oder Palladium, absorbiert auf einem Trägermaterial wie Aktivkohle, als Katalysator dienen, entfernt.

**II**

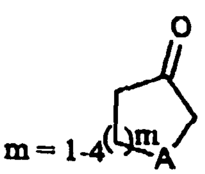

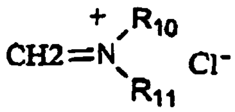

III

NHR10R11

IV

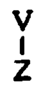

V

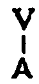

VI

Lithlumorganische Verbindungen der Formel **V**, in der Z Li und V eine G entsprechende Bedeutung hat, lassen sich durch Umsetzung von Halogenverbindungen der Formel **VI**, in der A gleich Cl, Br oder I bedeutet und V eine G entsprechende Bedeutung hat, mit beispielsweise n-Butyllithium/Hexan-Lösung durch Halogen-Lithiumaustausch erhalten. Man erhält so zunächst Produkte der allgemeinen Formel **VII**, in der $R^{10}$ eine $R^1$ analoge Bedeutung, $R^{11}$ eine $R^2$ analoge Bedeutung und V eine G entsprechende Bedeutung hat. Durch Umsetzung von Verbindungen der allgemeinen Formel **VII** mit Thionylchlorid und anschließender basischer Aufarbeitung erhält man die Verbindungen der Formel **IXb**. In manchen Fällen tritt ein Gemisch aus Verbindungen der allgemeinen Formel **VIII, IXa** und **IXb,** in der $R^{10}$ eine $R^1$ analoge Bedeutung, $R^{11}$ eine $R^2$ analoge Bedeutung und V eine G entsprechende Bedeutung hat. Diese können säulenchromatographisch oder durch Kristallisation getrennt werden. Gezielt können Verbindungen der allgemeinen Formel **IXa** durch Umsetzungen von Verbindungen der allgemeinen Formel **VII** mit Schwefelsäure erhalten werden.

Anschließende Hydrierung von Verbindungen der allgemeinen Formel **IXa** oder **IXb,** in der $R^{10}$ eine $R^1$ analoge Bedeutung, $R^{11}$ eine $R^2$ analoge Bedeutung und V eine G entsprechende Bedeutung hat, mit katalytisch aktiviertem Wasserstoff, wobei Platin oder Palladium, absorbiert auf einem Trägermaterial wie Aktivkohle, als Katalysator dienen, führt zu Verbindungen der Formel **X,** in der $R^{10}$ eine $R^1$ analoge Bedeutung, $R^{11}$ eine $R^2$ analoge Bedeutung und V eine G entsprechende Bedeutung hat. Die Hydrierung wird in einem Lösungsmittel wie Essigsäureethylester oder einem $C_1$ - $C_4$-Alkylalkohol bei Drücken von 0,1 bis 10 bar und Temperaturen von 20°C bis 80°C durchgerührt.
Ist A gleich S so können diese Verbindungen auch an geeigneter Stelle der Reaktionssequenz mit einem Oxidationsmittel in die entsprechenden SO bzw. $SO_2$ -Verbindungen überführt werden.

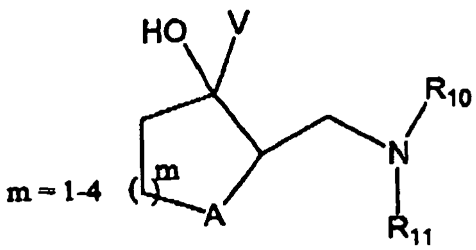

VII

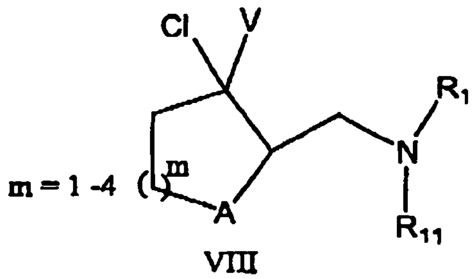

VIII

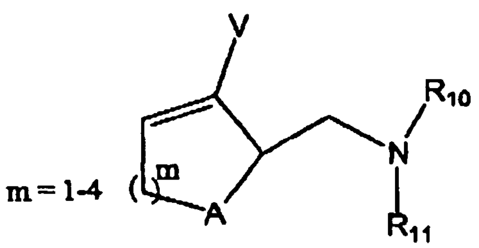

IXa

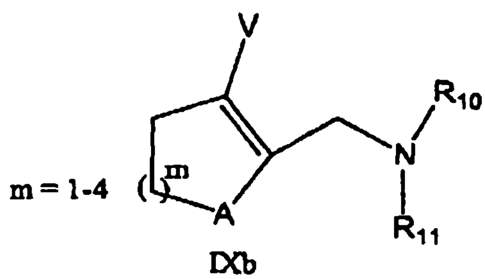

IXb

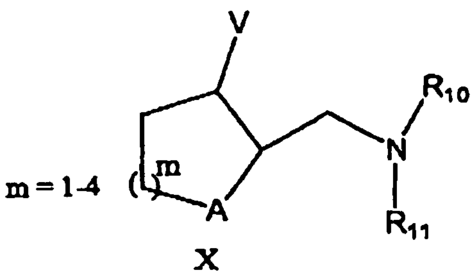

Unter den genannten Reaktionsbedingungen können OH- SH und $NH_2$-Gruppen unerwünschte Nebenreaktionen eingehen. Es ist daher bevorzugt, diese mit Schutzgruppen zu versehen oder im Falle von $NH_2$ durch $NO_2$ zu ersetzen und im letzten Reaktionsschritt die Schutzgruppe abzuspalten, bzw. die $NO_2$-Gruppe zu reduzieren. Ein weiterer Gegenstand der Erfindung ist daher eine Abwandlung des oben beschriebenen Verfahrens, bei dem mindestens eine in Formel **I** enthaltene OH-Gruppe durch eine $OSi(Ph)_2$tert.but.-Gruppe, mindestens eine SH-Gruppe durch eine S-p-Methoxybenzylgruppe und/oder mindestens eine $NH_2$-Gruppe durch eine $NO_2$-Gruppe ersetzt wird und nach Abschluß der gesamten Reaktionssequenz eine $OSi(Ph)_2$tert.but.-Gruppe mit Tetrabutylammoniumflluorid in Tetrahydrofuran und/oder mindestens eine p-Methoxybenzylgruppe mit einem Metallamin bevorzugt Natriumamin abgespalten und/oder mindestens eine $NO_2$-Gruppe zu $NH_2$ reduziert wird.

Weiter sind Carbonsäure- oder Thlocarbonsäure-Gruppen unter den Bedingungen der BuLi-Reaktion unter Umständen nicht stabil, so dass es bevorzugt ist, deren Methylester umzusetzen und das Verfahrensprodukt aus der BuLi-Reaktion im letzten Reaktionsschritt mit KOH-Lösung bzw. NaOH-Lösung in Methanol bei 40°C - 60°C zu verseifen. Ein weiterer Gegenstand der Erfindung ist daher eine Abwandlung der oben beschriebenen Verfahren, in dem nach der BuLi-Reaktion ein Verfahrensprodukt mit mindestens einer $C(O)OCH_3$- und/oder $C(S)OCH_3$-Gruppe mit KOH-Lösung bzw. NaOH-Lösung in Methanol bei 40°C - 60°C verseift wird.

Die Reinigung der in den einzelnen Reaktionssequenzen erhaltenen Verbindungen erfolgt durch Kristallisation oder Säulenchromatographie.

Die Verbindungen der Formel **I** lassen sich mit physiologisch verträglichen Säuren wie Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure in an sich bekannter Weise in ihre Salze überführen. Vorzugsweise wird die Salzbildung in einem Lösungsmittel wie Diisopropylether, Essigsäurealkylester, Aceton und/oder 2-Butanon durchgeführt. Zur Herstellung der Hydrochloride ist Trimethylchlorsilan in wasserhaltiger Lösung besonders geeignet.

Die erfindungsgemäßen gesättigten und ungesättigten Heteroarylcyctoalkylmethyl-Amine der allgemeinen Formel **I** sind toxikologisch unbedenklich und eignen sich daher als pharmazeutische Wirkstoffe in Arzneimitteln.

Ein weiterer Gegenstand der vorliegenden Erfindung sind daher Arzneimittel, die wenigstens eine erfindungsgemäße Verbindung der allgemeinen Formel **I** sowie gegebenenfalls physiologisch verträgliche Hilfsstoffe enthalten. Vorzugsweise eignen sich die erfindungsgemäßen Arzneimittel zur Bekämpfung von Schmerzen (insbesondere chronischer Schmerz, neuropathischer Schmerz, Entzündungsschmerz), Migräne, Fibromyalgia oder zur Behandlung von Depressionen (Unipolar, schwere Depression mit und ohne Wahn, mittelgradige Depressionen, leichte Depressionen, Melancholie, bipolare Depressionen; Bipolare Erkrankungen I (Manie und schwere Depressionen), Bipolare Erkrankungen II (Hypomanie und schwere Depressionen) Zyklothyme Persönlichkeitsstörungen (Hypomanie und milde Depressionen), Subtypen), Angstzuständen (Subtypen Geneiralisierte Angststörungen, Panikattacken, Zwangssyndromen, social anxiety disorder, Phobien, PSTD), Schlafstörungen, Harninkontinenz (Stress- und Drang-), Essstörungen, Bulemie, Attention deficit hyperactivity disorder, Sucht und Abhängigkeit, Trichotillomanie, Neuroleptikum sowie Gedächtnisstörungen.

Die Verwendung wenigstens einer Verbindung der allgemeinen Formel **I** zur Herstellung eines Arzneimittels zur Bekämpfung von Schmerzen (insbesondere chronischer Schmerz, neuropathischer Schmerz, Entzündungsschmerz), Migräne, Fibromyalgia oder zur Behandlung von Depressionen (Unipolar, schwere Depression mit und ohne Wahn, mittelgradige Depressionen, leichte Depressionen, Melancholie, bipolare Depressionen; Bipolare Erkrankungen I (Manie und schwere Depressionen), Bipolare Erkrankungen II (Hypomanie und schwere Depressionen) Zyklothyme Persönlichkeitsstörungen (Hypomanie und milde Depressionen), Subtypen), Angstzuständen (Subtypen Geneiralisierte Angststörungen, Panikattacken, Zwangssyndromen, social anxiety disorder, Phobien, PSTD), Schlafstörungen, Harninkontinenz (Stress- und Drang-), Essstörungen, Bulemie, Attention deficit hyperactivity disorder, Sucht und Abhängigkeit, Trichotillomanie, Neuroleptikum sowie Gedächtnisstörungen, ist ebenfalls Gegenstand der vorliegenden Erfindung.

Die erfindungsgemäßen Arzneimittel können als flüssige, halbfeste oder feste Arzneiformen, beispielsweise

in Form von Injektionslösungen. Tropfen, Säften, Sirupen, Sprays, Suspensionen, Tabletten, Patches, Kapseln, pflastern, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Neben wenigstens einer erfindungsgemäßen Verbindung der allgemeinen Formel **I** enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel **I** in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der allgemeinen Formel **I** auch verzögert freisetzen.

Die Herstellung der erfindungsgemäßen Arzneimittel kann mit Hilfe von üblichen, dem Fachmann bekannten Mitteln, Vorrichtungen, Methoden und Verfahren erfolgen, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen gesättigten oder ungesättigten Heteroarylcycloalkylmethylamine der allgemeinen Formel I kann variieren und ist beispielsweise abhängig vom Gewicht oder dem Alter des Patienten sowie von der Applikationsart, der Indikation und dem Schweregrad der Erkrankung. Üblicherweise werden 0,005 bis 500 mg/kg, vorzugsweise 0,05 bis 5 mg/kg Körpergewicht des Patienten wenigstens einer erfindungsgemäßen Verbindung der allgemeinen Formel **I** appliziert.

## Beispiele

Die folgenden Beispiele zeigen erfindungsgemäße Verbindungen sowie deren Darstellung und mit diesen durchgeführte Wirksamkeitsuntersuchungen.

Dabei gelten generell folgende Angaben:

Die Ausbeuten der hergestellten Verbindungen sind nicht optimiert.

Alle Temperaturen sind unkorrigiert.

Als stationäre Phase für die Säulenchromatographie wurde Kieselgel 60 (0.040 - 0.063 mm) der Firma E. Merck, Darmstadt, eingesetzt.

Die dünnschichtchromatographischen Untersuchungen wurden mit HPTLC-Fertigplatten. Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Die Angabe Ether bedeutet Diethylether.

Soweit nicht anders angegeben, wurde Petrolether mit dem Siedebereich von 50°C-70°C benutzt.

### *Beispiel 1:*

Dimethyl-(2-pyridin-3-yl-cyclohex-1-enylmethyl)amin Dihydrochlorid

Zu einer Lösung von 54 ml 3-Bompyridin in 750 ml Diethylether p.a. wurden innerhalb von 30 Minuten bei einer Temperatur von -35 bis -40 °C 224 ml n-Butyllithiumlösung (2,5 mol/l in Hexan) zugetropft. Nach weiteren 20 Minuten bei dieser Temperatur wurden bei fortgesetzter Kühlung 86,9 g 2-Dimethylaminomethylcyclohexanon, gelöst in 90 ml Diethylether p.a., innerhalb von 30 Minuten zugetropft und nochmals 60 Minuten unter leichter Erwärmung auf -30 °C gerührt. Anschließend wurden unter Erwärmung des Reaktionsansatzes auf 0 °C 150 ml Wasser zugegeben, die Phasen getrennt, die wässrige Phase zweimal mit Ethylacetat extrahiert, die vereinigten organischen Phasen Ober Magnesiumsulfat getrocknet, filtriert und eingeengt. Das erhaltene Rohprodukt (139 g) wurde in 700 ml absolutem Ethanol gelöst, nacheinander mit 142 ml Chlortrimethylsilan und 20,2 ml Wasser versetzt und 12 Stunden im Eisbad gerührt. Der

ausgefallene Feststoff wurde abgesaugt, mit wenig absolutem Ethanol gewaschen und für zwei Stunden bei 90 °C im Vakuum (ca. 50 mbar) getrocknet. Es wurden 63,9 g 2-Dimethylaminomethyl-1-pyridin-3-yl-cyclohexanol Hydrochlorid erhalten.

Zu 74,9 g 2-Dinnethylaminomethyl-l-pyridin-3-yl-cyclohexanol Hydrochlorid wurden unter starker Gasentwicklung 129 ml Thionylchlorid zugetropft, die Mischung unter Rühren für 2,5 Stunden auf 60 bis 65 °C erhitzt und anschließend an der Wasserstrahlpumpe eingeengt. Nach Abkühlung wurden zunächst 200 ml Wasser, dann 100 ml Natronlauge (32 massenprozentig) zugegeben, anschließend zweimal mit je 250 ml eines Gemisches gleicher Volumina von Tetrahydrofuran und Ethylacetat extrahiert, die vereinigten Extrakte über Magnesiumsulfat getrocknet, filtriert und eingeengt. Das erhaltene Rohprodukt (48,9 g) wurde in 350 ml absolutem Ethanol gelöst, nacheinander mit 57,4 ml Chlortrimethylsilan und 8,1 ml Wasser versetzt und 12 Stunden im Eisbad gerührt Der ausgefallene Feststoff wurde abgesaugt, mit wenig absolutem Ethanol gewaschen und für zwei Stunden bei 90 °C im Vakuum (ca. 50 mbar) getrocknet Es wurden 34,7 g rohes Dimethyl-(2-pyridin-3-yl-cyclohex-1-enylmethyl)amin Dihydrochlorid erhalten. Zur Endreinigung wurden 168 g von nach obiger Vorschrift hergestelltem rohen Dimethyl-(2-pyridin-3-yl-cyclohex-1-enylmethyl)amin Dihydrochlorid in 650 ml siedendem absolutem Ethanol gelöst und unter Rühren durch Kühlung im Eisbad zur erneuten Kristallisation gebracht. Nach Rühren über Nacht bei Raumtemperatur wurde abgesaugt, mit wenig absolutem Ethanol gewaschen und für 4,5 Stunden bei 120 °C im Vakuum (ca. 50 mbar) getrocknet. Es wurden 121 g Dimethyl-(2-pyridin-3-yl-cyc; lohex-l-enylmethyl)-amin Dihydrochlorid mit einem Schmelzpunkt von 215 bis 218 °C erhalten.

Analog zu Beispiel 1 wurden hergestellt:

***Beispiel 23:*** Dimethyl-(2-pyridln-3-yl-cyclohept-1-enylmethyl)-amin Hydrochlorid

***Beispiel 28:*** Dlmethyl-(2-pyridin-3-yl-cyclopent-1-enylmethyl)-amin Hydrochlorid

***Beispiel 32:*** Dimethyl-(2-pyridin-3-yl-cyclooct-1-enylrnethyl)-amin Hydrochlorid

***Beispiel 38:*** Dimethyl-(2-pyridin-4-yl-cyclohept-1-enylmethyl)-amin Hydrochlorid

***Beispiel 39:*** Dimethyl-(2-pyridin-4-yl-cyclohex-1-enylmethyl)-Hydrochlorid

***Beispiel 42:*** Dimethyl-(2-pyridin-4-yl-cyclooct-1-enylmethyl)-amin Hydrochlorid

***Beispiel 47:*** Dimethyl-(2-pyridin-4-yl-cyclopent-1-enylmethyl)-amin Hydrochlorid

***Beispiel 54:*** [2-(5-Methoxy-pyridin-3-yl)-cyclohex-1-enylmethyl]-dimethyt-amin Hydrochlorid

***Beispiel 64:*** 5-(2-Dimathylaminomethyl-cyclohex-1-enyl)-pyridin-3-Hydrochlorid

***Beispiel 65:*** Dipropyl-(2-pyridin-3-yl-cyclohex-1-enylmethyl)-Hydrochlorid

***Beispiel 68:*** [2-(6-Chlor-pyridin-3-yl)-cyclohex-1-enylmethyl]-dimethylamin Hydrochlorid

***Beispiel 74:*** [2-(5-Methoxy-pyridin-3-yl)-cyclooct-1-enylmethyl]-dimethyl-amin Hydrochlorid

***Beispiel 76:*** 5-(2-Dimethylaminomethyl-cyclohept-1-enyl)-pyridin-3-Hydrochlorid.

Teilweise fielen bei der Herstellung der vorgenannten Verbindungen auch regloisomere Olefine an:

***Beispiel 20:*** Gemisch aus [2-(4-Brom-6-chlor-pyridin-3-yl)-cyclohex-1-enylmethyl]-methyl-amln Hydrochlorid und [2-(4-Brom-6-chlor-pyridin-3-yl)-cyclohex-2-enytmethyl]-methyl-amin Hydrochlorid

***Beispiel 25:*** Gemisch aus [2-(6-Chlor-pyridin-3-yl)-cyclooct-1-enylmethyl]-dimethyl-amin Hydrochlorid und [2-(6-Chlor-pyridin-3-yl)-cyclooct-2-enylmethyl]-dimethyl-amin Hydrochlorid

***Beispiel 40:*** Gemisch aus Dimethyl-(2-pyridin-4-yl-cyclohex-1-enylmethyl)-amin Hydrochlorid und Dimethyl-(2-pyridin-4-yl-cyclohex-2-enylmethyl)-amin Hydrochlorid

***Beispiel 58:*** Gemisch aus [2-(5-Methoxy-pyridin-3-yl)-cyclohept-1-enylmethyl]-dimethyl-amin Hydrochlorid und [2-(5-Methoxy-pyridin-3-yl)-cyclohept-2-enylmethyl]-dimethyl-amin Hydrochlorid

***Beispiel 59:*** Gemisch aus 5-(2-Dimethylaminomethyl-cyclohept-1-enyl)-pyridin-3-ol; Hydrochlorid und 5-(7-Dimethylaminomethylcyclohept-1-enyl)-pyridin-3-ol; Hydrochlorid

***Beispiel 67:*** Gemisch aus [2-(5-Methoxy-pyridin-3-yl)-cyclooct-1-enylmethyl]-dimethyl-amin Hydrochlorid und [2-(5-Methoxy-pyridin-3-yl)-cyclooct-2-enylmethyl]-dimethyl-amin Hydrochlorid

Neben den Olefinen fielen für die nachfolgenden Beispiele nach der in Beispiel 1 beschriebenen Vorgehensweise auch die nachfolgenden chlorierten Derivate an, die säulenchromatographisch von den Olefinen abgetrennt wurden:

***Beispiel 13:*** [2-Chlor-2-(6-chlor-pyridin-3-yl)-cycloheptylmethyl]-dimethyl-amin Hydrochlorid

***Beispiel 14:*** (2-Chloro-2-pyridin-3-yl-cyclohexylmethyl)-dimethyl-Hydrochlorid

***Beispiel 15:*** [2-Chlor-2-(6-chlor-pyridin-3-yl)-cyclohexylmethyl]-dimethylamin Hydrochlorid

***Beispiel 36:*** (2-Chlor-2-pyridin-4-yl-cycloheptylmethyl)-dimethyl-amin Hydrochlorid.

***Beispiel 2:***

Methyl-(2-pyridin-3-yl-cyclohex-1-enylmethyl)amin Hydrochlorid

37,6 ml Cyclohexanon, 5,75 g Paraformaldehyd, 38 ml Eisessig und 30,2 g Benzylmethylamin Hydrochlorid wurden unter Rühren für 25 Minuten auf 105 °C (Badtemperatur) erhitzt, der Ansatz im Vakuum (10 mbar) eingeengt, der Rückstand in 100 ml 2-Butanon aufgenommen und nochmals im Vakuum eingeengt. Der Rückstand wurde mit 150 ml 2-Butanon zum Rückfluss erhitzt. Nach Abkühlung auf Raumtemperatur wurde filtriert und der Filterrückstand im Vakuum getrocknet. Es wurden 36 g 2-[(Benzylmethylamino)methyl]cyclohexanon erhalten.

Zu einer Lösung von 12,1 ml 3-Brompyridin in 180 ml Diethylether p.a. wurden innerhalb von 30 Minuten bei einer Temperatur von -45 bis -50 °C 50 ml n-Butyllithiumlösung (2,5 mol/l in Hexan) zugetropft. Nach weiteren 20 Minuten bei dieser Temperatur wurden bei fortgesetzter Kühlung 25,2 g 2-[(Benzylmethylamino)methyl]cyciohexanon, gelöst in 50 ml Diethylether p.a., innerhalb von 30 Minuten zugetropft und nochmals 60 Minuten nachgerührt. Anschließend wurde die Kühlung entfernt und unter Erwärmung des Reaktionsansatzes von -20 auf 0 °C 54 ml Wasser zugegeben, die Phasen getrennt, die wässrige Phase zweimal mit einem Gemisch gleicher Volumina von Tetrahydrofuran und Ethylacetat extrahiert, die vereinigten organischen Phasen über Magnesiumsulfat getrocknet, filtriert und eingeengt. Das erhaltene Rohprodukt (33,0 g) wurde zunächst mit Aceton/Ethylacetat (V:V = 1:2), anschließend nochmals mit reinem Ethylacetat an Kieselgel chromatographiert, die erhaltene Produktfraktion (23,6 g) in 230 ml Ethylacetat gelöst, mit 2,74 ml Wasser und 19,3 ml Chlortrimethylsilan versetzt und die Mischung, aus der sich ein Öl absetzte, zur Trockne eingeengt. Der Rückstand wurde in 90 ml Wasser und 17,9 ml Natronlauge (32 massenprozentig) aufgenommen, zweimal mit je 90 ml Ethylacetat extrahiert, die vereinigten Extrakte über Magnesiumsulfat getrocknet, filtriert und eingeengt. Es wurden erneut 23,6 g Rohprodukt erhalten, die, in 230 ml Aceton gelöst, mit 2,74 ml Wasser und 19,3 ml Chlortrimethylsilan, gelöst in 100 ml Aceton, versetzt wurden. Der Überstand wurde vom gebildeten Feststoff abdekantiert und der Rückstand mit 230 ml Aceton auf 50 °C erwärmt. Nach Abkühlung wurde erneut abdekantiert und der Rückstand im Vakuum (50 mbar) bei 50 °C getrocknet. Es wurden 28,8 g 2-[(Benzylmethylamino)methyl]-1-pyridin-3-yl-cyclohexanol Hydrochlorid erhalten.

11,1 g 2-[(Benzylmethylamino)methyl]-1-pyridin-3-yl-cyclohexanol Hydrochlorid wurden unter Stickstoff in 110 ml Eisessig gelöst, als Katalysator 2,0 g Palladium auf Aktivkohle (10 massenprozentig) zugegeben und unter Rühren bei 2,0 bar Wasserstoffdruck bis zum Ende der Wasserstoffaufnahme gerührt. Der Ansatz wurde abgesaugt, das Filtrat eingeengt, der Rückstand mit 75 ml Isopropanol über Nacht gerührt und der erhaltene Niederschlag abgesaugt und getrocknet. Es wurden 6,18 g 2-Methylaminomethyl-1-pyridin-3-yl-cyclohexanol Hydrochlorid erhalten.

Zu 5,20 g 2-Methylaminomethyl-1-pyridin-3-yl-cyclohexanol Hydrochlorid wurden 26 ml Thionylchlorid zugetropft, die Mischung unter Rühren für 90 Minuten erhitzt (Badtemperatur 65 °C) und anschließend an der Wasserstrahlpumpe eingeengt. Nach Abkühlung wurde verdünnte Natronlauge (2 mol/l) zugegeben, mit einem Gemisch gleicher Volumina von Tetrahydrofuran und Ethylacetat wiederholt extrahiert, die vereinigten Extrakte über Magnesiumsulfat getrocknet, filtriert und eingeengt. Das erhaltene Rohprodukt (3,25 g) wurde in 25 ml absolutem Ethanol und 8 ml Ethylacetat gelöst und nacheinander mit 4,1 ml Chlortrimethylsilan und 0,58 ml Wasser versetzt. Der ausgefallene Feststoff (1,84 g) wurde abgesaugt (vgl. Beispiel 79), die Mutterlauge mit verdünnter Natronlauge (2 mol/l) basisch gestellt, mit einem Gemisch gleicher Volumina von Tetrahydrofuran und Ethylacetat wiederholt extrahiert, die vereinigten Extrakte über Magnesiumsulfat getrocknet, filtriert und eingeengt. Der erhaltene Rückstand (1,45 g) wurde mit Methanol,

dem 0,05 Volumenprozent wässriger Ammoniaklösung (25 massenprozentig) zugesetzt wurden, an Kieselgel chromatographiert. Die Produktfraktion (0,52 g) wurde in 50 ml Ethylacetat gelöst und 0,66 ml Chlortrimethylsilan gefolgt von 92 µl Wasser zugegeben. Als Niederschlag wurden 0,57 g Methyl-(2-pyridin-3-yl-cyclohex-1-enylmethyl)amin Hydrochlorid erhalten.

**Beispiel 79:**

Methyl-(2-pyridin-3-yl-cyclohex-2-enylmethyl)amin Hydrochlorid

Wie für Beispiel 2 beschrieben wurden 1,84 g Methyl-(2-pyridin-3-ylcyclohex-2-enylmethyl)amin erhalten, die wie dort beschrieben in Aceton mit Wasser und Chlortrimethylsilan in das korrespondierende Hydrochlorid überführt wurden.

***Beispiel 4:***

Dimethyl-(2-pyridin-3-yl-cyclohex-2-enylmethyl)amin Hydrochlorid

Zu einer Lösung von 54 ml 3-Bompyridin in 750 ml Diethylether p.a. wurden innerhalb von 30 Minuten bei einer Temperatur von -35 bis -40 °C 224 ml n-Butyllithiumlösung (2,5 mol/l in Hexan) zugetropft. Nach weiteren 20 Minuten bei dieser Temperatur wurden bei fortgesetzter Kühlung 86,9 g 2-Dimethylaminomethylcyclohexanon, gelöst in 90 mi Diethylether p.a., innerhalb von 30 Minuten zugetropft und nochmals 60 Minuten unter leichter Erwärmung auf -30 °C gerührt. Anschließend wurden unter Erwärmung des Reaktionsansatzes auf 0 °C 150 ml Wasser zugegeben, die Phasen getrennt, die wässrige Phase zweimal mit Ethylacetat extrahiert, die vereinigten organischen Phasen über Magnesiumsulfat getrocknet, filtriert und eingeengt. Das erhaltene Rohprodukt (139 g) wurde in 700 mi absolutem Ethanol gelöst, nacheinander mit 142 ml Chlortrimethylsilan und 20,2 ml Wasser versetzt und 12 Stunden im Eisbad gerührt. Der ausgefallene Feststoff wurde abgesaugt, mit wenig absolutem Ethanol gewaschen und für zwei Stunden bei 90 °C im Vakuum (ca. 50 mbar) getrocknet. Es wurden 63,9 g 2-Dimethylaminomethyl-1-pyridin-3-yl-cyclohexanol Hydrochlorid erhalten.

Zu 7,8 g Dimethylaminomethyl-1-pyridin-3-yl-cyclohexanol Hydrochlorid wurden unter Eiskühlung 40 ml 96%-ige Schwefelsäure gegeben. Nach Beendigung der Gasentwicklung wurde der Ansatz 45 Minuten lang bei Raumtemperatur gerührt. Anschließend wurde die Lösung auf ca. 400 g zerstoßenes Eis gegossen. Dann wurde unter Eiskühlung durch Zugabe von Natriumhydroxidplätzchen alkalisiert und das Gemisch 3 x mit insgesamt 600 ml Essigester extrahiert. Die vereinigten organischen Phasen wurden aber Magnesiumsulfat getrocknet und bei 50°C Im Vakuum einrotiert. Man erhielt so 4,2 g Dimethyl-(2-pyridin-3-yl-cyclohex-2-enylmethyl)amin Hydrochlorid.

Analog wurden hergestellt

***Beispiel 24:*** Dimethyl-(2-pyridin-3-yl-cyclohept-2-enylmethyl)-Hydrochlorid

***Beispiel 29:*** Dimethyl-(2-pyridin-3-yl-cyclopent-2-enylmethyl)-amin Hydrochlorid

***Beispiel 33:*** Diniethyl-(2-pyridin-3-yl-cyclooct-2-enylmethyl)-amin Hydrochlorid

***Beispiel 37:*** Dimethyl-(2-pyridin-4-yl-cyclohept-2-enylmethyl)-amin Hydrochlorid.

***Beispiel 43:*** Dimethyl-(2-pyridin-4-yl-cyclopent-2-enylmethyl)-amin Hydrochlorid

***Beispiel 48:*** [2-(6-Chlor-pyridin-3-yl)-cyclohept-2-enylmethyl]-dimethyl-amin Hydrochlorid

***Beispiel 49:*** [2-(6-Chlor-pyridin-3-yl)-cyclohex-1-enylmethyl]-dimethylamin Hydrochlorid

***Beispiel 55:*** [2-(5-Methoxy-pyrldin-3-yl)-cyclohex-2-enylmethyl]-dimethyl-amin Hydrochlorid

***Beispiel 66:*** Dipropyl-(2-pyridin-3-yl-cyclohex-2-enylmethyl)-amin Hydrochlorid

***Beispiel 69:*** [2-(6-Chlor-pyridin-3-yl)-cyclohex-2-enylmethyl]-dimethylamin Hydrochlorid

***Beispiel 71:*** [2-(6-Chlor-pyridin-3-yl)-cyclooct-2-enylmethyl]-dimethylamin Hydrochlorid

***Beispiel 73:*** [2-(5-Methoxy-pyridin-3-yl)-cyclooct-2-enylmethyl]-dimethyl-amin Hydrochlorid

***Beispiel 75:*** 5-(7-Dimethylaminomethyl-cyclohept-1-enyl)-pyridin-3-Hydrochlorid.

***Beispiel 6:***

cis-Dimethyl-(2-pyridin-3-yl-cyclohexylmethyl)amin Hydrochlorid

6,90 g Dimethyl-(2-pyridin-3-yl-cyclohex-2-enylmethyl)amin Hydrochlorid wurden in 70 ml Methanol gelöst und in Gegenwart von 1,04 g Palladium auf Aktivkohle (10 massenprozentig) für fünf Stunden bei 2 bar Wasserstoffdruck hydriert. Anschließend wurde filtriert, das Filtrat Im Vakuum eingeengt, der Rückstand mit zweimolarer Natronlauge und einer Mischung gleicher Volumina Ethylacetat und Tetrahydrofuran in die Base überführt, die mit Ethylacetat extrahiert wurde. Nach Trocknung und Einengen wurde der Rückstand (4,47 g) mit einer Mischung gleicher Volumina Methanol, Ethylacetat und n-Hexan an Kieselgel chromatographiert. Die Hauptfraktion (3,72 g) wurde in ca. 40 ml Aceton/Ethanol (V/V =9:1) gelöst und mit einem halben Moläquivalent Wasser und einen Moläquivalent Chlortrimethylsilan versetzt. Nach Rühren über Nacht wurde zur Trockne eingeengt, der Rückstand mit 72 ml THF und 18 ml Ethanol für 30 Minuten im Ultraschallbad behandelt, die Suspension über Nacht gerührt, abgesaugt und der Rückstand getrocknet. Es wurden 2,42 g cis-Dimethyl-(2-pyridin-3-yl-cyclohexylmethyl)amin Hydrochlorid mit einen Schmelzpunkt von 219-222 °C erhalten.

***Beispiel 7:***

trans-Dimethyl-(2-pyridin-3-yl-cyclohexylmethyl) Hydrochlorid

Zu einer Lösung von 54 ml 3-Bompyridin in 750 ml Diethylether p.a. wurden innerhalb von 30 Minuten bei einer Temperatur von -35 bis -40 °C 224 ml n-Butyllithiumlösung (2,5 mol/l in Hexan) zugetropft. Nach weiteren 20 Minuten bei dieser Temperatur wurden bei fortgesetzter Kühlung 86,9 g 2-Dimethylaminomethylcyclohexanon, gelöst in 90 ml Diethylether p.a., innerhalb von 30 Minuten zugetropft und nochmals 60 Minuten unter leichter Erwärmung auf -30 °C gerührt. Anschließend wurden unter Erwärmung des Reaktionsansatzes auf 0 °C 150 ml Wasser zugegeben, die Phasen getrennt, die wässrige Phase zweimal mit Ethylacetat extrahiert, die vereinigten organischen Phasen über Magnesiumsulfat getrocknet, filtriert und eingeengt. Das erhaltene Rohprodukt (139 g) wurde in 700 mi absolutem Ethanol gelöst, nacheinander mit 142 ml Chlortrimethylsilan und 20,2 ml Wasser versetzt und 12 Stunden im Eisbad gerührt. Der ausgefallene Feststoff wurde abgesaugt, mit wenig absolutem Ethanol gewaschen und für zwei Stunden bei 90 °C im Vakuum (ca. 50 mbar) getrocknet. Es wurden 63,9 g 2-Dimethylaminomethyl-1-pyridin-3-yl-cyclohexanol Hydrochlorid erhalten.

Zu 7,8 g Dimethylaminomethyl-1-pyridin-3-yl-cyclohexanol Hydrochlorid wurden unter Eiskühlung 40 ml 96%-Ige Schwefelsäure gegeben. Nach Beendigung der Gasentwicklung wurde der Ansatz 45 Minuten lang bei Raumtemperatur gerührt. Anschließend wurde die Lösung auf ca. 400 g zerstoßenes Eis gegossen. Dann wurde unter Eiskühlung durch Zugabe von Natriumhydroxidplätzchen alkalisiert und das Gemisch 3 x mit insgesamt 600 ml Essigester extrahiert. Die vereinigten organischen Phasen wurden über Magnesiumsulfat getrocknet und bei 50°C im Vakuum einrotiert. Man erhielt so 4,2 g. der Titelverbindung.

3.0 g Dimethyl-(2-pyridin-3-yl-cyclohex-2-enylmethyl)-amin Hydrochlorid wurden in Ethanol (100 ml) gelöst und anschließend mit Palladium auf Kohle (10% Pd, 300 mg) versetzt. Die Suspension wurde für 4 Stunden bei einem Druck von 4 bar mit Wasserstoff (0,821) hydriert. Anschließend wurde der Katalysator durch Filtration abgetrennt und das Lösungsmittel am Rotationsverdampfer abdestilliert. Es wurden 3,1 g eines weißen hygroskopischen Feststoffs erhalten. Das Rohprodukt wurde durch Alkalisieren mit Natronlauge und nachfolgender Extraktion mit Ethylacetat in die Base überführt. Anschließend wurde es zweimal an Kieselgel chromatographiert (Diisopropylether : Methanol : Ammoniak = 300 : 150 : 1 und Acetonitril : Ethanol : Ammoniak = 200:100:1). Das erhaltene Rohprodukte (70 mg) wurde in Ethylacetat gelöst (20 ml). Anschließend wurde unter Eiskühlung Trimethylchlorsilan (0,08 ml; 0,63 mmol) und Wasser (0,01 ml; 0,55 mmol) zugegeben und für 30 Minuten nachgerührt. Nach Filtration wurde das Dimethyl-(2-pyridin-3-yl-cyclohexylmethyl)-amin Hydrochlorid (0,09 g) als weißer Feststoff erhalten.

In Analogie zu Beispiel 7 wurden hergestellt:

***Beispiel 26:*** cis-Dimethyl-(2-pyridin-3-yl-eycloheptylmethyl)-amin Hydrochlorid

***Beispiel 27:*** trans-Dimethyl-(2-pyridin-3-yl-cycloheptylmethyl)-amin Hydrochlorid

***Beispiel 31:*** Dimethyl-(2-pyridin-3-yl-cyclopentylmethyl)-amin Hydrochlorid

***Beispiel 35:*** Dimethyl-(2-pyridin-3-yl-cyclooctylmethyl)-amin Hydrochlorid

***Beispiel 60:*** [2-(5-Methoxy-pyridin-3-yl)-cycloheptylmethyl]-dimethylamin Hydrochlorid

***Beispiel 62:*** [2-(5-Methoxy-pyridin-3-yl)-cyclohexylmethyl]-dimethyl-Hydrochlorid

***Beispiel 63:*** Gemisch aus cis-[2-(5-Methoxy-pyridin-3-yl)-cyclohexylmethyl]-dimethyl-amin Hydrochlorid und trans-[2-(5-Methoxy-pyridin-3-yl)-cyclohexylmethyl]-dimethyl-amln Hydrochlorid

***Beispiel* 70:** trans-[2-(5-Methoxy-pyridin-3-yl)-cyclooctylmethyl]-dimethyl-amin und das entsprechende Hydrochlorid

***Beispiel 72:*** cis-[2-(5-Methoxy-pyridin-3-yl)-cyclooctylmethyl]-dimethyl-amin Hydrochlorid

***Beispiel 77:*** 5-(2-Dimethylaminomethyl-cycloheptyl)-pyridin-3-ol Hydrochlorid.

***Beispiel 10:***

Benzylmethyl-(2-pyridin-3-yl-cyclohex-1-enylmethyl)amin Hydrochlorid

5,5 g 2-[(Benzylmethylamino)methyl]-1-pyridin-3-ylcyclohexanol Hydrochlorid und 27,5 ml Thionylchlorid wurden für eine Stunde bei einer Badtemperatur von 75 °C erhitzt. Nach Einengen im Vakuum wurde der Rückstand mit Ethylacetat und Natronlauge (32 massenprozentig) aufgenommen, die organische Phase abgetrennt, getrocknet und eingeengt. Das erhaltene Rohprodukt (3.7 g) wurde mit DIisopropylether/Ethylacetat (V/V = 2:1) an Kieselgel chromatographiert. Es wurden 1,23 g Benzylmethyl-(2-pyridin-3-ylcyclohex-1-enylmethyl)amin erhalten, die in ca. 25 ml Aceton gelöst und mit 1,66 µl Wasser und 1.18 ml Chlortrimethylsilan in das korrespondierende Hydrochlorid überführt wurden.

***Beispiel 11:***

Benzylmethyl-(2-pyridin-3-yl-cyclohex-2-enylmethyl)amin Hydrochlorid

Wie für **Beispiel 10** beschrieben wurden auch 1,37 g Benzylmethyl-(2-pyridin-3-yl-cyclohex-2-enylmethyl)amin erhalten, die in ca. 30 ml Aceton gelöst und mit 1,86 µl Wasser und 1,30 ml Chlortrimethylsilan in das korrespondierende Hydrochlorid überführt wurden.

***Beispiel 50:***

cis-3-Dimethylaminomethyl-4-pyridin-3-yl-tetrahydrothiopyran-4-ol Hydrochlorid

5,00 g Tetrahydrothiopyran-4-on wurden in 15 ml Acetonitril gelöst und 4,03 g N,N-Dimethyl-methylenammoniumchlorid gefolgt von 40 µl Acetylchlorid zugegeben. Nach 45 Minuten Rühren bei 60 °C Badtemperatur wurde 60 Stunden bei Raumtemperatur gerührt, der gebildete Niederschlag abgesaugt und getrocknet. Es wurden 8,17 g 3-Dimethylaminomethyltetrahydrothiopyran-4-on Hydrochlorid mit einen Schmelzpunkt von 155-157 °C erhalten. Hieraus wurde mit Dichlormethan und Natronlauge (32 massenprozentig) die korrespondierende Base freigesetzt.

Zu einer Lösung von 7,5 ml 3-Bompyridin in 110 ml Diethylether p.a. wurden innerhalb von 15 Minuten bei einer Temperatur von -40 bis -45 °C 31 ml n-Butyllithiumlösung (2,5 mol/l in Hexan) zugetropft. Nach weiteren 60 Minuten bei - 50 °C wurden bei fortgesetzter Kühlung 10,8 g 3-Dimethylaminomethyltetrahydrothiopyran-4-on, gelöst in 45 ml Diethylether p.a., innerhalb von 15 Minuten zugetropft und über Nacht unter Erwärmung auf Raumtemperatur gerührt. Bei ca 15 °C wurden 25 ml Wasser zugegeben, die Phasen getrennt, die wässrige Phase zweimal mit Diethylether extrahiert, die vereinigten organischen Phasen über Magnesiumsulfat getrocknet, filtriert und eingeengt. Das erhaltene Rohprodukt (15,0 g) wurde mit einer Mischung gleicher Volumina Methanol, Ethylacetat und n-Hexan an Kieselgel chromatographiert. Die Hauptfraktion (7,83 g) wurde in ca. 80 ml Ethanol gelöst und mit 2,2 Moläquivalenten Chlortrimethylsilan und Wasser in das Hydrochlorid von cis-3-Dimethylaminomethyl-4-pyridin-3-yl-tetrahydrothiopyran-4-ol mit einen Schmelzpunkt von 262-263 °C überführt.

***Beispiel 51:***

trans-3-Dimethylaminomethyl-4-pyridin-3-yl-tetrahydrothiopyran-4-ol Hydrochlorid

Wie für **Beispiel 50** beschrieben wurden bei der Säulenchromatographie als Nebenprodukt auch 1,03 g des polareren Diastereoisomeren trans-3-Dimethylaminomethyl-4-pyridin-3-yltetrahydrothiopyran-4-ol Hydrochlorid erhalten, die analog in das korrespondierende Hydrochlorid überführt wurden.

***Beispiel 52:***

cis-3-Dimethylaminomethyl-4-pyridin-3-yl-tetrahydropyran-4-ol Hydrochlorid

9,6 g Tetrahydropyran-4-on wurden in 29 ml Acetonitril gelöst und 9,69 g N,N-Dimethyl-mothylenammoniumchlorid gefolgt von 100 µl Acetylchlorid zugegeben. Nach 45 Minuten Rühren bei 60 °C Badtemperatur wurde 60 Stunden bei Raumtemperatur gerührt, der gebildete Niederschlag abgesaugt und getrocknet Es wurden 16,4 g 3-Dimethylaminomethyltetrahydropyran-4-on Hydrochlorid erhalten. Hieraus wurde mit Dichlormethan und Natronlauge (32 massenprozentig) die korrespondierende Base freigesetzt.

Zu einer Lösung von 8,4 ml 3-Bompyridin in 125 ml Diethylether p.a. wurden innerhalb von 15 Minuten bei einer Temperatur von -40 bis -45 °C 35 ml n-ButyUithiumlösung (2,5 mol/l in Hexan) zugetropft. Nach weiteren 60 Minuten bei - 50 °C wurden bei fortgesetzter Kühlung 8,7 g 3-Dimethylaminomethyltetrahydropyran-4-on, gelöst in 45 ml Diethylether p.a., innerhalb von 15 Minuten zugetropft und über Nacht unter Erwärmung auf Raumtemperatur gerührt. Bei ca 15 °C wurden 28 ml Wasser zugegeben, die Phasen getrennt, die wässrige Phase zweimal mit Diethylether extrahiert, die vereinigten organischen Phasen über Magnesiumsulfat getrocknet, filtriert und eingeengt. Das erhaltene Rohprodukt (13,5 g) wurde mit einer Mischung gleicher Volumina Methanol und Dichlormethan an Kieselgel chromatographiert. Neben einer kleinen Menge (1,5 g) des polareren trans-Diastereoisomers wurde die Hauptfraktion (3,70 g) In ca. 40 ml Ethanol gelöst und mit 2,2 Moläquivalenten Chlortrimethylsilan und Wasser in das Hydrochlorid von cis-3-Dimethylaminomethyl-4-pyridin-3-yltetrahydropyran-4-ol Hydrochlorid mit einen Schmelzpunkt von 276-277 °C überführt.

***Beispiel 53:***

Dimethy-(4-pyridin-3-yl-5,6-dihydro-2H-pyran-3-yl-methyl)amin Hydrochlorid

4,95 g eines Gemisches von cis- und trans-3-Dimethylaminomethyl-4-pyridin-3-yl-tetrahydropyran-4-ol Hydrochlorid und 6,6 ml Thionylchlorid wurden für zwei Stunden bei einer Badtemperatur von 65 °C und anschließend über Nacht bei Raumtemperatur gerührt. Nach Einengen im Vakuum wurde der Rückstand mit Ethylacetat und Natronlauge (32 massenprozentig) aufgenommen, die organische Phase abgetrennt, getrocknet und eingeengt. Das erhaltene Rohprodukt (2,2 g) wurde mit einem Gemisch gleicher Volumina Methanol und Dichlormethan an Kieselgel chromatographiert. Neben 0,82 g des Zielprodukts wurde eine Mischfraktion von 1,26 g erhalten, die nochmals unter analogen Bedingungen chromatographiert wurde. Insgesamt wurden so 0,96 g des Zielprodukts erhalten, die in ca. 10 ml Ethanol gelöst und mit 0,18 ml Wasser und 1,23 ml Chlortrimethylsilan in das korrespondierende Hydrochlorid von Dimethyl-(4-pyridin-3-yl-5,6-dihydro-2H-pyran-3-yl-methyl)amin überführt mit einen Schmelzpunkt von 244-248 °C wurden.

**Pharmakologische Untersuchungen**

**a) Untersuchungen der 5-HT- und NA-Wiederaufnahmehemmung**

Um diese in vitro Studien durchführen zu können, werden Synaptosomen aus Rattenhirnarealen frisch isoliert. Es findet jeweils eine sogenannte "$P_2$"-Fraktion Verwendung, die exakt nach der Vorschrift von Gray, E.G. und Whittaker, V.P. (1962, J. Anat. 76, 79-88) präpariert wird. Für die NA-Wiederaufnahme werden diese vesikulären Partikel aus dem Hypothalamus, für die 5-HT-Wiederaufnahme aus der Medulla + Pons-Region von männlichen Rattengehirnen isoliert.

Folgende Kenndaten wurden für die NA- und 5-HT-Wiederaufnahme ermittelt:

| | |
|---|---|
| NA-Wiederaufnahme: | Km = 0,32 ± 0,11 µM |
| 5-HT-Wiederaufnahme: | Km = 0.084 ± 0,011 µM |

(Jeweils N = 4, d.h. Mittelwerte ± SEM aus 4 unabhängigen Versuchsreihen, die In Dreifach-Parallelversuchen durchgeführt wurden).

Eine detaillierte Methodenbeschreibung ist in der Publikation von Frink, M.Ch., Hennies, H.-H., Englberger, W. et al. (1996, Arzneim.-Forsch./Drug Res. 46 (III), 11, 1029-1036) enthalten (der Ansatz kann auch auf Mikrotiterplatten (250 μl / well) bei Zimmertemperatur durchgeführt werden).

Auswertungen:

Neben % Hemmungen bei fixen Testsubstanzkonzentrationen (z.B. 1 x $10^{-6}$ M oder 1 x $10^{-5}$ M im Ansatz), wurden Dosisabhängigkeiten überprüft. Hierbei werden $IC_{50}$-Werte erhalten, die gemäß der "Cheng-Prusoff Gleichung" (Cheng, Y.C. und Prusoff, W.H., 1973. Biochem. Pharmacol. 22, 3099-3108) in Inhibitorkonstanten ($K_i$) umgerechnet werden können. Die $IC_{50}$ Werte wurden mit Hilfe des Computer-Programms "Figure P" (Version 6.0. Biosoft, Cambridge. England) erhalten. Km-Werte wurden gemäß Lineweaver, H. und Burk, D. (1934, J. Am. Chem. Soc. 56, 658-666) berechnet. Um $K_D$-Werte darzustellen, ist das Computer-Programm "Ligand" (Version 4, Biosoft, England) angewendet worden.

Für die erfindungsgemäßen Verbindungen wurde eine deutliche Hemmung der Serotonin- oder Noradrenalinwiederaufnahme gemessen. Die Ergebnisse ausgewählter Untersuchungen der 5-HT- und NA-Wiederaufnahmehemmung im Vergleich zu Venlafaxin und Duloxetin sind In der nachfolgenden Tabelle 1 dargestellt.

Tabelle 1

| Verbindung gemäß Beispiel | 5-HT-Wiederaufnahmehemmung (10 μmol/l) | NA-Wiederaufnahmehemmung (10 μmol/l) |
|---|---|---|
| 1 | 83 % | 35 % |
| 2 | 69% | 15% |
| 6 | 94% | 25% |
| 7 | 95% | 22 % |
| 13 | 38% | 75% |
| 14 | 73% | 27% |
| 15 | 51% | 53% |
| 20 | 82% | 74 % |
| 23 | 86 % | 50 % |
| 24 | 67% | 19 % |
| 25 | 47% | 71% |
| 26 | 82 % | 28% |
| 27 | 88 % | 54% |
| 28 | 83% | 29% |
| 29 | 69% | 64% |
| 31 | 70% | 8% |
| 32 | 78% | 55% |
| 33 | 74% | 50% |
| 38 | 76% | 73% |
| 40 | 79% | 42% |
| 48 | 81% | 82% |
| 49 | 55% | 67% |
| 50 | 54% | 29% |
| 54 | 85% | 17% |
| 55 | 67% | 34% |

(fortgesetzt)

| Verbindung gemäß Beispiel | 5-HT-Wiederaufnahmehemmung (10 µmol/l) | NA-Wiederaufnahmehemmung (10 µmol/l) |
|---|---|---|
| 58 | 84% | 47 % |
| 59 | 53% | 48% |
| 60 | 75% | 30% |
| 62 | 77% | 13% |
| 63 | 78% | 18% |
| 64 | 68% | 6% |
| 65 | 54% | -8% |
| 67 | 78% | 59% |
| 68 | 67% | 62% |
| 70 | 89% | 52% |
| 71 | 74 % | 77 % |
| 72 | 60% | 53 % |
| 73 | 89% | 55 % |
| 74 | 72% | 56% |
| Venlafaxin | 82% | 92 % |
| Duloxetin | 88 % | 87% |

**b) Untersuchungen der analgetischen Wirkungen im Formalin-Test an der Maus**

Der Formalin Test (Dubuisson, D. and Dennis, S.G., 1977, Pain, 4, 161-174) stellt ein Modell für den akuten sowie den chronischen Schmerz dar. In den hier vorgestellten Untersuchungen wurde die chronische Schmerzkomponente ausgewertet.

Durch eine einmalige Formalln-Injektion in die dorsale Seite einer Hinterpfote wird bei freibeweglichen Versuchstieren eine biphasische nociceptive Reaktion induziert, die durch Beobachtung von drei deutlich voneinander unterscheidbaren Verhaltensmustern erfasst wird.

Formalin wird mit dem Volumen von 20 µl und einer Konzentration von 1 % subkutan in die dorsale Seite der rechten Hinterpfote jedes Tieres appliziert. Die vom Normalverhalten (Score 0) differierenden spezifischen Verhaltensänderungen, wie Anheben (Score 1) und SchUttein der Pfote (Score 2), sowie Beiss- und Leckreaktionen (Score 3) werden bis 60 min nach Formalinapplikation in Teilintervallen von 3 min kontinuierlich beobachtet und registriert. Die Verhaltensänderungen werden unterschiedlich gewichtet (Score 0-3) und eine Pain-Rate (PR) errechnet mit folgender Formel:

$$PR = [(T_0 \times 0) + (T_1 \times 1) + (T_2 \times 2) + (T_3 \times 3) / 180.$$

Dabei entsprechen $T_0$, $T_1$, $T_2$, $T_3$ jeweils der Zeit in Sekunden, in der das Tier die Verhaltensweisen 0, 1. 2, oder 3 zeigte. Die Gruppengröße beträgt 10 Tiere (n=10).

Basierend auf den PR-Berechnungen wurde die Substanzwirkung als Änderung gegen eine Kontrolle in Prozent ermittelt. Die $ED_{50}$-Bestimmung erfolgte mittels Regressionsanalyse.

Für die Verbindung gemäß Beispiel 1 wurde eine dosisabhängige Hemmung des nociceptiven Verhaltens beobachtet. Die Ergebnisse sind in Tabelle 2 dargestellt.

Tabelle 2

| Formalin-Test an der Maus | |
|---|---|
| **Verbindung gemäß Beispiel** | **Hemmung des nociceptiven Verhaltens versus Kontrolle; $ED_{50}$-Wert bzw. % Hemmung** |
| 1 | 1,66 mg/kg i. v. |
| 2 | 88 % (21,5 mg/kg i. v.) |
| 7 | 2.55 mg/kg i. v. |
| 10 | 66,3 % (10 mg/kg i. v.) |
| 20 | 66,5 % (10 mg/kg i. v.) |
| 23 | 2,44 mg/kg i. v. |
| 24 | 5,4 mg/kg i. v. |
| 28 | 92,6 % (10 mg/kg i. v.) |
| 31 | 89,7 % (10 mg/kg i. v.) |
| 32 | 2,77 mg/kg i. v. |
| 50 | 37,0% (21,5 mg/kg i. v.) |
| 65 | -39,4 % (4,64 mg/kg i. v.) |
| Venlafaxin | 2.60 mg/kg i. v. |
| Duloxetin | 6,69 mg/kg i. p. |

**c) Untersuchung der antidepressiven Wirkung im Forced swimming-Test (Porsolt-Test) an der Maus**

Die Untersuchungen zur Bestimmung der antidepressiven Wirkung der erfindungsgemäßen Verbindungen der Formel I wurden im Forced swimming -Test (Porsolt-Test) an der Maus durchgeführt (Porsolt, R. et al., Arch. Int. Pharmacodyn. Vol. 229, S. 327-336, (1977)). Männliche Mäuse (20-25 g Körpergewicht) wurden individuell für eine Dauer von 6 Minuten in ein flaches Wasserbecken gesetzt, aus dem sie nicht entfliehen konnten und somit gezwungen waren zu schwimmen. Nach einiger Zeit gaben die Tiere ihre Schwimmversuche auf und gingen in eine Immobilitätsphase über. In dem Intervall von 2 - 6 Minuten nach dem Einbringen des Tieres wurde Dauer der Immobilitätsphase bestimmt. Substanz- und Vehikelgruppen umfassen jeweils 10 Tiere. Änderungen der Dauer der Immobilitätsphase werden relativ zur Vehikelkontrolle angegeben. Unterschiede versus Vehikelkontrolle werden mit Hilfe des Student's T-Test auf Signifikanz geprüft. Antidepressiva induzieren eine Verkürzung der Immobilitätsphase.
Für die Verbindung gemäß Beispiel 1 wurde eine signifikante Verkürzung der Immobilitätsphase und somit eine antidepresslve Wirkung im Forced swimming-Test ermittelt. Die Ergebnisse sind in Tabelle 3 dargestellt.

Tabelle 3

| Forced swimming-Test an der Maus | | | |
|---|---|---|---|
| **Änderung der Immobilitätsphase versus Kontrolle** | | | |
| **Dosis** | **Verbindung gemäß Beispiel 1** | **Venlafaxin** | **Duloxetin** |
| 21,5 mg/kg i. p. | -54 %*** | -66 %*** | -41 %** |
| Student's T-Test; ***: $p < 0,001$ | | | |

**d) Untersuchung der anxiolytischen Wirkung im Isolatlon-Induced Pup Vocallsation-Test an der Maus**

Zur Untersuchung von anxiolytischen Wirkungen wurde der Isolation-induced Pup vocalisation-Test an der Maus verwendet (Fish, E.W., Sekinda, M., Ferrari, P. F., Dirks, A., Miczek, K. A., Psychopharmacology, Vol. 149, S. 277-285, (2000)). Für die Testung wurden Würfe von 7 bis 13 Mäusen verwendet. Im Alter von 7 Tagen wurden die Tiere von der Mutter getrennt und in einen Inkubationskäfig mit einer Innentemperatur von ca. 34°C gesetzt. Zur Bestimmung der Vokalisationen im Ultraschallbereich wurden die Tiere einzeln auf eine temperaturkontrollierte Plattform

(23cm x 23 cm, durch ein Raster unterteilt In Felder von 2cm x 2cm, 19±1°C) einer schallgeschützten Testkammer für die Dauer von 30 sec gesetzt. Die Ultraschall-Vokalisationen wurden mit einem Hochfrequenz-Kondensatormikrofon aufgenommen, gefiltert, amplifiziert digitalisiert und unter Verwendung kommerzieller Software aufgezeichnet und analysiert. Für die Testung wurden nur Tiere mit Vorwerten von mindestens 6 Vokalisationen innerhalb von 30 sec und einem Körpergewicht von 3,5 bis 5,5 g eingesetzt. Nach subkutaner (s. c.) Substanz- oder Vehikelinjektion wurden die Tiere für die Dauer von 10 min in den Inkubationskäfig zurückgesetzt und anschließend erneut einzeln auf die temperaturkontrollierte Platte der Testkammer für die Dauer von 4 min plaziert. Zusätzlich zur Anzahl der Vokalisationen wurden Überquerungen des Rasters der Platte sowie Körperdrehungen der Mäuse zur Untersuchung von Substanzeinflüssen auf die lokomotorische Aktivität bzw. Koordination bestimmt. Änderungen der Vokalisatlonsfrequenzen, Rasterüberquerungen und Körperdrehungen wurden relativ zur Vehikelkontrolle angegeben. Für Anxiolytika ist eine Reduktion der Vokalisatlonsfrequenz beschrieben.

Für die Verbindung gemäß Beispiel 1 wurde eine signifikante Reduktion der Vokalisationsfrequenz gemessen. Nach Gabe dieser Verbindung wurden keine signifikanten Veränderungen der Rasterüberquerungen und Körperdrehungen beobachtet. Somit wurde eine selektive anxiolytische Wirkung ohne Beeinflussung der Lokomotion und Koordination im Isolation-induced Pup vocalisation-Test ermittelt. Die Ergebnisse für die Verbindung gemäß Beispiel 1 sowie für Venlafaxin und Citalopram sind in den drei nachfolgenden Tabellen dargestellt.

Tabelle 4

| **Änderung der Vokalisationsfrequenz versus Kontrolle** | | | |
|---|---|---|---|
| | **Verbindung gemäß Beispiel 1** | **Venlafaxin** | **Citalopram** |
| $ED_{50}$-Wert | 1,7 mg/kg s. c. | 8.1 mg/kg s. c. | 1,2 mg/kg s. c. |

Tabelle 5

| **Änderung der Rasterüberquerungen** | | | |
|---|---|---|---|
| | **Verbindung gemäß Beispiel 1** | **Venlafaxin** | **Citalopram** |
| **Dosisbereich** | 1,0-17 mg/kg s. c. | 0,3-56 mg/kg s. c. | 0,56-10 mg/kg s. c. |
| **Maximale Änderung** | +32% (n. s.) | +92 % (s.) | +66% (n. s.) |
| ANOVA, post-hoc Dunnett's Test ($\alpha$= 0.05); n. s.: nicht signifikant; s.: signifikant (p<0,05) | | | |

Tabelle 6

| **Änderung der Körperdrehungen** | | | |
|---|---|---|---|
| | **Verbindung gemäß Beispiel 1** | **Venlafaxin** | **Citalopram** |
| **Dosisbereich** | 1,0 -17 mg/kg s. c. | 0,3-56 mg/kg s. c. | 0,56-10 mg/kg s. c. |
| **Maximale Änderung** | + 233 % (n. s.) | +141% (n. s.) | + 397 % (s.) |
| ANOVA, post-hoc Dunnett's Test ($\alpha$= 0.05); n. s.: nicht signifikant; s.: signifikant (p<0,05) | | | |

**Patentansprüche**

1. Gesättigte und ungesättigte Heteroarylcycloalkylmethyl-Amine der allgemeinen Formel **I**

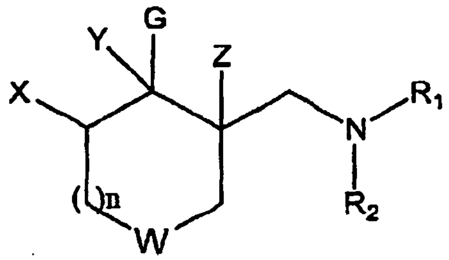

worin

W gleich $CH_2$ ist, wobei Y ausgewählt ist aus H oder Cl und gleichzeitig X und Z gleich H sind
oder
X und Y bilden zusammen eine Bindung und Z ist gleich H
oder
Y und Z bilden zusammen eine Bindung und X ist gleich H,

oder

W gleich O, S. SO oder $SO_2$ ist, wobei Y ausgewählt ist aus H, OH, Cl und X und Z gleich H sind
oder
X und Y bilden zusammen eine Bindung und Z ist gleich H
oder
Y und Z bilden zusammen eine Bindung und X ist gleich H,

und n = 0 - 3 ist,

$R^1$ und $R^2$ unabhängig voneinander ausgewählt sind aus H, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH. $OCH_3$ oder unsubstituiert; $C_3$-$C_7$-Cycloalkyl, gesättigt oder ungesättigt, einfach oder mehrfach substituiert durch F. Cl, Br, I, $NH_2$, SH oder OH, $OCH_3$ oder unsubstituiert, bzw. einem entsprechenden Heterocyclus, bei dem ein C-Atom im Ring durch S, O oder $NR^3$ ersetzt ist, mit $R^3$ ausgewählt aus
H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert;
Alkylaryl, gesättigt oder ungesättigt, einfach oder mehrfach substituiert durch F, Cl, Br, I, $NH_2$, SH oder OH, $OCH_3$ oder unsubstituiert; Alkylheteroaryl, gesättigt oder ungesättigt, einfach oder mehrfach substituiert durch F, Cl, Br, I, $NH_2$, SH oder OH, $OCH_3$ oder unsubstituiert, Aryl, einfach oder mehrfach substituiert durch F, Cl, Br, I, $NH_2$, SH oder OH, $OCH_3$ oder unsubstituiert;

oder

$R^1$ und $R^2$ zusammen ein $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bilden, bzw. einen entsprechenden Heterocyclus, bei dem ein C-Atom im Ring durch S, O oder $NR^4$ ersetzt ist,
mit $R^4$ ausgewählt aus
H, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert,

und
G ausgewählt ist aus Pyridin-3-yl oder Pyridin-4-yl und unsubstituiert oder einfach oder mehrfach substituiert ist, wobei mindestens ein Wasserstoffrest ersetzt ist durch F, Cl, Br, I, CN, $CF_3$. $NO_2$, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert; $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bzw. einem entsprechenden Heterocyclus, bei dem ein C-Atom im Ring durch S,
O oder $NR^5$ ersetzt ist, mit $R^5$ ausgewählt aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert;

$OR^6$, $OC(O)R^6$, $OC(S)R^6$, $C(O)R^6$, $C(O)OR^6$, $C(S)R^6$, $C(S)OR^6$, $SR^6$, $S(O)R^6$ bzw. $S(O_2)R^6$, wobei $R^6$ ausgewählt ist aus

H, $C_1$-$C_{10}$-Alkyl, $C_2$-$C_{10}$-Alkenyl oder $C_2$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert; $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bzw. einem entsprechenden Heterocyclus, bei dem ein C-Atom im Ring durch S, O oder $NR^7$ ersetzt ist, mit $R^7$ ausgewählt aus H, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert;

Alkylaryl, gesättigt oder ungesättigt, einfach oder mehrfach substituiert durch F, Cl, Br, I, $NH_2$, SH oder OH, $OCH_3$ oder unsubstituiert; Aryl oder Heteroaryl, jeweils einfach oder mehrfach substituiert durch F, Cl, Br, I, $NH_2$, SH oder OH, $OCH_3$ oder unsubstituiert;

$NR^8R^9$ $C(O)NR^8R^9$ oder $S(O_2)NR^8R^9$, wobei $R^8$ und $R^9$ unabhängig voneinander ausgewählt sind aus

H, $C_1$-$C_{18}$-Alkyl, $C_2$-$C_{18}$-Alkenyl oder $C_3$-$C_{18}$-Alkinyl, jeweils verzweigt oder unverzweigt, einfach oder mehrfach substituiert durch F, Cl, Br, I, $NH_2$, SH oder OH, $OCH_3$ oder unsubstituiert; $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bzw. einem entsprechenden Heterocyclus, bei dem ein C-Atom im Ring durch S, O oder $NR^{10}$ ersetzt ist, mit $R^{10}$ ausgewählt aus H, $C_1$-$C_{10}$-Alkyl, $C_2$-$C_{10}$-Alkenyl oder $C_3$-$C_{10}$-Alkinyt, jeweils verzweigt oder unverzweigt, einfach oder mehrfach substituiert durch F, Cl, Br, I, $NH_2$, SH oder OH, $OCH_3$ oder unsubstituiert;

Alkylaryl, gesättigt oder ungesättigt, einfach oder mehrfach substituiert durch F, Cl. Br, I, $NH_2$, SH oder OH, $OCH_3$ oder unsubstituiert; Aryl oder Heteroaryl, jeweils einfach oder mehrfach substituiert durch F, Cl, Br, I, $NH_2$, SH oder OH, $OCH_3$ oder unsubstituiert;

oder

$R^8$ und $R^9$ zusammen ein $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bilden, bzw. einen entsprechenden Heterocyclus, bei dem ein C-Atom im Ring durch S, O oder $NR^{10}$ ersetzt ist, mit $R^{10}$ ausgewählt aus

H, $C_1$-$C_{10}$-Alkyl, $C_2$-$C_{10}$-Alkenyl oder $C_3$-$C_{10}$-Alkinyl, jeweils verzweigt oder unverzweigt, einfach oder mehrfach substituiert durch F, Cl, Br, I, $NH_2$, SH oder OH, $OCH_3$ oder unsubstituiert:

Alkyiaryl, Aryl oder Heteroaryl, jeweils einfach oder mehrfach substituiert durch F, Cl, Br, I, $NH_2$, SH oder OH, $OCH_3$ oder unsubstituiert,

auch in Form ihrer Razemate; Enantiomere. Diastereomere, insbesondere Mischungen ihrer Enantiomere oder Diastereomere oder eines einzelnen Enantiomers oder Diastereomers sowie ihrer freien Basen oder eines mit einer physiologisch verträglichen Säure gebildeten Salzes, insbesondere des Hydrochloridsalzes.

2. Verbindungen der allgemeinen Formel **I** nach Anspruch 1, **dadurch gekennzeichnet, dass** W gleich $CH_2$ und n = 0 - 3 ist.

3. Verbindungen der allgemeinen Formel **I** nach Anspruch 1, **dadurch gekennzeichnet, dass** W gleich O, S, SO oder $SO_2$ ist und n = 0 - 3 ist.

4. Verbindungen der allgemeinen Formel **I** nach Anspruch 3, **dadurch gekennzeichnet, dass** W gleich O oder S ist und n = 0 - 3 ist.

5. Verbindungen der allgemeinen Formel **I** nach einem der Ansprüche 1 bis 4, **dadurch gekennzeichnet, dass**

   $R_1$ und $R_2$ unabhängig voneinander ausgewählt sind aus H, $C_1$-$C_{10}$-Alkyl, Alkylaryl, gesättigt oder ungesättigt, einfach oder mehrfach substituiert durch F, Cl, Br, I, $NH_2$, SH oder OH, $OCH_3$ oder unsubstituiert; Alkylheteroaryl, gesättigt oder ungesättigt, einfach oder mehrfach substituiert durch F, Cl, Br, I, $NH_2$, SH oder OH, $OCH_3$ oder unsubstituiert,

   oder

   $R_1$ und $R_2$ zusammen ein $C_3$-$C_7$-Cycloalkyl, gesättigt oder ungesättigt, einfach oder mehrfach substituiert durch F, Cl, Br, I, $NH_2$, SH oder OH, $OCH_3$ oder unsubstituiert, bilden,

   und der Rest G unsubstituiert oder durch Cl, Br oder $OR_6$, wobei $R_8$ gleich H oder $C_1$-$C_{10}$-Alkyl ist, substituiert ist.

**6.** Verbindungen der allgemeinen Formel **I** nach einem der Ansprüche 1, 2 und 5, wobei die Verbindungen ausgewählt sind aus:

Dimethyl-(2-pyridln-3-yl-cyclohex-1-enylmethyl)-amin und das entsprechende Dihydrochlorid **(1)**
Methyl-(2-pyridin-3-yl-cyclohex-1-enylmethyl)-amin und das entsprechende Hydrochlorid **(2)**
Dimethyl-(2-pyridin-3-yl-cyclohex-2-enylmethyl)amin und das entsprechende Hydrochlorid **(4)**
Dimethyl-(2-pyridin-3-yl-cyclohexytmethyl)-amin und das entsprechende Hydrochlorid; Diasteroisomer 1 **(6)**
Dimethyl-(2-pyridin-3-yl-cyclohexylmethyl)-amine und das entsprechende Hydrochlorid; Diastereoisomer 2 **(7)**
Benzyl-methyl-(2-pyridin-3-yl-cyclohex-1-enylmethyl)-amin und das entsprechende Hydrochlorid **(10)**
Benzyl-methyl-(2-pyridin-3-yl-cyclohex-2-enylmethyl)-amin und das entsprechende Hydrochlorid **(11)**
[2-Chlor-2-(6-chlor-pyridin-3-yl)-cycloheptylmethyl]-dimethyl-amin und das entsprechende Hydrochlorid **(13)**
(2-Chloro-2-pyridin-3-yl-cyclohexylmethyl)-dimethyl-amin und das entsprechende Hydrochlorid **(14)**
[2-Chlor-2-(6-chlor-pyridin-3-yl)-cyclohexylmethyl]-dimethylamin und das entsprechende Hydrochlorid **(15)**
Gemisch [2-(4-Brom-6-chlor-pyridin-3-yl)-cyclohex-1-enylmethyl]-methylamin und [2-(4-Brom-6-chlor-pyridin-3-yl)-cyclohex-2-enylmethyl]-methylamin und die entsprechenden Hydrochloride **(20)**
Dimethyl-(2-pyridin-3-yl-Cyclohept-1-enylmethyl)-amin und das entsprechende Hydrochlorid **(23)**
Dimethyl-(2-pyridin-3-yl-cyclohept-2-enylmethyl)-amin und das entsprechende Hydrochlorid **(24)**
Gemisch aus [2-(6-Chlor-pyridin-3-yl)-cyclooct-1-enylmethyl]-dimethylamin Hydrochlorid und [2-(6-Chlor-pyridin-3-yl)-cyclooct-2-enylmethyl]-dimethyl-amin Hydrochlorid **(25)**
Dimethyl-(2-pyridin-3-yl-cycloheptylmethyl)-amin und das entsprechende Hydrochlorid; Diastereoisomer 1 **(26)**
Dimethyl-(2-pyridin-3-yl-cycloheptylmethyl)-amln und das entsprechende Hydrochlorid; Diastereoisomer 2 **(27)**
Dimethyl-(2-pyridin-3-yl-cyclopent-1-enylmethyl)-amin und das entsprechende Hydrochlorid **(28)**
Dimethyl-(2-pyridin-3-yhcyclopent-2-enylmethyl)-amin und das entsprechende Hydrochlorid **(29)**
Dimethyl-(2-pyridin-3-yl-cyclopentylmethyl)-amin und das entsprechende Hydrochlorid **(31)**
Dimethyl-(2-pyridin-3-yl-cyclooct-1-enylmethyl)-amin und das entsprechende Hydrochlorid **(32)**
Dimethyl-(2-pyridin-3-yl-cyclooct-2-enylmethyl)-amin und das entsprechende Hydrochlorid **(33)**
Dimethyl-(2-pyridin-3-yl-cyctooctylmethyl)-amin und das entsprechende Hydrochlorid **(35)**
(2-Chlor 2-pyridin-4-yl-cycloheptylmethyl)-dirnethyt-amin und das entsprechende Hydrochlorid **(36)**
Dimethyl-(2-pyridin-4-yl-cyclohept-2-enytmethyl)-arnin und das entsprechende Hydrochlorid **(37)**
Dimethyl-(2-pyridin-4-yl-cyclohept-1-enytmethyl)-amin und das entsprechende Hydrochlorid **(38)**
Dimethyl-(2-pyridin-4-yl-cyclohex-1-enylmethyt)-amin und das entsprechende Hydrochlorid **(39)**
Gemisch aus Dimethyl-(2-pyridin-4-yl-cyclohex-1-enylmethyl)-amin Hydrochlorid und Dimethyl-(2-pyridin-4-yl-cyclohex-2-enylmethyl)-amin Hydrochlorid **(40)**
Dimethyl-(2-pyridin-4-yl-cyclooct-1-enylmethyl)-amin und das entsprechende Hydrochlorid **(42)**
Dimethyl-(2-pyridin-4-yl-cyclopent-2-enylmethyl)-amin und das entsprechende Hydrochlorid **(43)**
Dimethyl-(2-pyridin-4-yl-cyclopent-1-enytmethyl)-amine und das entsprechende Hydrochlorid **(47)**
[2-(6-Chlor-pyridin-3-yl)-cyclohept-2-enylmethyl]-dimethyl-amin und das entsprechende Hydrochlorid **(48)**
[2-(6-Chlor-pyridin-3-yl)-cyclohex-1-enylmethyl]-dimethyl-amin und das entsprechende Hydrochlorid **(49)**
[2-(5-Methoxy-pyrtdin-3-yl)-cyclohex-1-enylmethyl]-dimethyl-amin und das entsprechende Hydrochlorid **(54)**
[2-(5-Methoxy-pyridin-3-yl)-cyclohex-2-enylmethyl]-dimethyl-amin und das entsprechende Hydrochlorid **(55)**
Gemisch aus [2-(5-Methoxy-pyridin-3-yl)-cyclohept-1-enylmethyl]-dimethyl-amin Hydrochlorid und [2-(5-Methoxy-pyridin-3-yl)-cyclohept-2-enylmethyl]-dimethyl-amin Hydrochlorid **(58)**
Gemisch aus 5-(2-Dimethylaminomethyl-cyclohept-1-enyl)-pyridin-3-ol; Hydrochlorid und 5-(7-Dimethylaminomethyl-cyclohept-1-enyl)-pyridin-3-ol; Hydrochlorid **(59)**
[2-(5-Methoxy-pyndin-3-yl)-cycloheptylmethyl]-dimethyl-amin und das entsprechende Hydrochlorid **(60)**
[2-(5-Methoxy-pyridin-3-yl)-cyclohexylmethyl]-dimethyl-amin und das entsprechende Hydrochlorid **(62)**
Gemisch aus 2-(5-Methoxy-pyridin-3-yl)-cyclohexylmethyl]-dimethyl-amin (Diastereoisomer 1) und [2-(5-Methoxy-pyridin-3-yl)-cyclohexylmethyl]-dimethyl-amin (Diastereoisomer 2) und den entsprechenden Hydrochloriden **(63)**
5-(2-Dimethyfaminomethyl-cyclohex-1-enyl)-pyridin-3-ol und das entsprechende Hydrochlorid **(64)**
Dipropyl-(2-pyridin-3-yl-cyclohex-1-enylmethyl)-amin und das entsprechende Hydrochlorid **(65)**
Dipropyl-(2-pyridin-3-yl-cyclohex-2-enylmethyl)-amin und das entsprechende Hydrochlorid **(66)**
[2-(5-Methoxy-pyridin-3-yl)-cyclooct-1-enylmethyl]-dimethyl-amin Hydrochlorid und [2-(5-Methoxy-pyridin-3-yl)-cyclaoct-2-enylmethyl]-dimethyl-amin und die entsprechenden Hydrochloride **(67)**
[2-(6-Chlor-pyridin-3-yl)-cyclohex-1-enylmethyl]-dimethyl-amin und das entsprechende Hydrochlorid **(68)**
[2-(6-Chlor-pyridin-3-yl)-cyclohex-2-enylmethyl]-dimethyl-amin und das entsprechende Hydrochlorid **(69)**
[2-(5-Methoxy-pyridin-3-yl)-cyclooctylmethyl]-dimethyl-amin und das entsprechende Hydrochlorid; Diastereoisomer 1 **(70)**

[2-(6-Chlor-pyridin-3-yl)-cyclooct-2-enylmethyl]-dimethyl-amin und das entsprechende Hydrochlorid **(71)**
[2-(5-Methoxy-pyridin-3-yl)-cyclooctylmethyl]-dimethyl-amin und das entsprechende Hydrochlorid; Diastereisomer 2 **(72)**
[2-(5-Methoxy-pyridin-3-yl)-cyclooct-2-enylmethyl]-dimethyl-amin und das entsprechende Hydrochlorid **(73)**
[2-(5-Methoxy-pyridin-3-yl)-cyclooct-1-enylmethyl]-dimethyl-amin und das entsprechende Hydrochlorid **(74)**
5-(7-Dimethylamlnomethyl-cyclohept-1-enyl)-pyridin-3-ol und das entsprechende Hydrochlorid **(75)**
5-(2-Dimethytaminomethyl-cyclohept-1-enyl)-pyridin-3-ol und das entsprechende Hydrochlorid **(76)**
5-(2-Dimethylaminomethyl-cycloheptyl)-pyridin-3-ol und das entsprechende Hydrochlorid **(77)**
Methyl-(2-pyridin-3-yl-cyclohex-2-enylmethyl)-amin und das entsprechende Hydrochlorid **(79).**

**7.** Verbindung der allgemeinen Formel **I** nach einem der Ansprüche 1, 3, 4 und 5, wobei die Verbindungen ausgewählt sind aus:

3-Dimethytaminomethyl-4-pyridin-3-yl-tetrahydro-thiopyran-4-ol und das entsprechende Hydrochlorid; Diastereoisomer 1 **(50)**
3-Dimethylaminomethyl-4-pyridin-3-yl-tetrahydro-thiopyran-4-ol und das entsprechende Hydrochlorid; Diastereoisomer 2 **(51)**
3-Dimethylaminomethyl-4-pyridin-3-yl-tetrahydro-pyran-4-ol und das entsprechende Hydrochlorid **(52)**
Dimethyl-(4-pyridin-3-yl-5,6-dihydro-2H-pyran-3-yl-methyl)-amin und das entsprechende Hydrochlorid **(53).**

**8.** Verfahren zur Herstellung von Verbindungen der allgemeinen Formel **I** sowie ihrer pharmazeutisch annehmbaren Salze, **dadurch gekennzeichnet, dass** Cycloalkanone der allgemeinen Formel **II** mit Immoniumsalzen der Formel **III** bzw. mit Paraformaldehyd und einem Amin der Formel **IV** umgesetzt werden, wobei $R^{10}$ eine $R^1$ analoge Bedeutung und $R^{11}$ eine $R^2$ analoge Bedeutung hat, die erhaltenen Mannich-Basen mit einer metallorganischen Verbindung der Formel **V**, in der Z U und V eine G entsprechende Bedeutung hat, in Lösungsmittel zwischen -70°C und 60°C umgesetzt werden, wobei die lithiumorganische Verbindung der Formel **V**, in der Z Li und V eine G entsprechende Bedeutung hat, durch Umsetzung von Halogenverbindungen der Formel **VI.** in der A gleich Cl, Br oder I bedeutet und V eine G entsprechende Bedeutung hat, mit vorzugsweise n-Butyllithium/Hexan-Lösung durch Halogen-Lithiumaustausch hergestellt wird, wodurch Produkte der allgemeinen Formel **VII**, in der $R^{10}$ eine $R^1$ analoge Bedeutung, $R^{11}$ eine $R^2$ analoge Bedeutung und V eine G entsprechende Bedeutung hat, erhalten werden, die mit Thionylchlorid umgesetzt und anschließend basisch zu einem Gemisch aus Verbindungen der allgemeinen Formeln **VIII, IXa** und **IXb**, in denen $R^{10}$ eine $R^1$ analoge Bedeutung, $R^{11}$ eine $R^2$ analoge Bedeutung und V eine G entsprechende Bedeutung hat, aufgearbeitet wird, die Verbindungen getrennt werden, anschließend die Hydrierung der Verbindungen der allgemeinen Formeln **Ixa** und **IXb** mit katalytisch aktiviertem Wasserstoff, wobei Platin oder Palladium, absorbiert auf einem Trägermaterial, als Katalysator dienen, in Lösungsmittel bei Drücken von 0,1 bis 10 bar und Temperaturen von 20°C bis 80°C zu Verbindungen der Formel **X**, in der $R^{10}$ eine $R^1$ analoge Bedeutung, $R^{11}$ eine $R^2$ analoge Bedeutung und V eine G entsprechende Bedeutung hat, durchgeführt wird.

**9.** Verfahren nach Anspruch 8, **dadurch gekennzeichnet, dass** mindestens eine in Formel **I** enthaltene OH-Gruppe durch eine $OSi(Ph)_2$tert.but.-Gruppe, mindestens eine SH-Gruppe durch eine S-p-Methoxybenzylgruppe und/oder mindestens eine $NH_2$-Gruppe durch eine $NO_2$-Gruppe ersetzt wird und nach Abschluß der gesamten Reaktionssequenz eine $OSi(Ph)_2$tert.but.-Gruppe mit Tetrabutylammoniumfluorid in Tetrahydrofuran und/oder mindestens eine p-Methoxybenzylgruppe mit einem Metallamin bevorzugt Natriumamin abgespalten und/oder mindestens eine $NO_2$-Gruppe zu $NH_2$ reduziert wird.

**10.** Verfahren nach den Ansprüchen 8 und 9, **dadurch gekennzeichnet, dass** nach der BuLi-Reaktion ein Verfahrensprodukt mit mindestens einer $C(O)OCH_3$- und/oder $C(S)OCH_3$-Gruppe mit KOH-Lösung bzw. NaOH-Lösung in Methanol bei 40°C - 60°C verseift wird.

**11.** Arzneimittel, enthaltend mindestens eine Verbindung der allgemeinen Formel **I** oder eines ihrer pharmazeutisch annehmbaren Salze.

**12.** Verwendung einer Verbindung der allgemeinen Formel **I** oder eines ihrer pharmazeutisch annehmbaren Salze zur Herstellung eines Arzneimittels zur Behandlung von Depressionen, Angststörungen und/oder Schmerzen.

**13.** Pharmazeutische Zusammensetzung, die mindestens eine Verbindung der allgemeinen Formel **I** oder eines ihrer pharmazeutisch annehmbaren Salze sowie mindestens einen pharmazeutischen Hilfsstoff enthält.

## Claims

1. Saturated and unsaturated heteroarylcycloalkylmethylamines of the general formula I

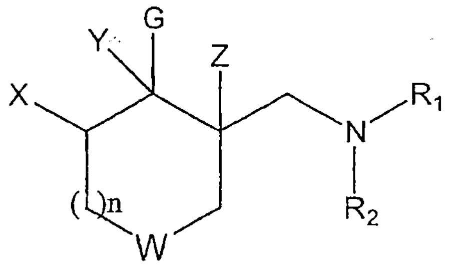

wherein

W is $CH_2$, wherein Y is chosen from H or Cl and at the same time X and Z are H
or
X and Y together form a bond and Z is H
or
Y and Z together form a bond and X is H,

or

W is O, S, SO or $SO_2$, wherein Y is chosen from H, OH or Cl and X and Z are H
or
X and Y together form a bond and Z is H
or
Y and Z together form a bond and X is H,

and n = 0 - 3,

$R^1$ and $R^2$ independently of one another are chosen from H, $C_1$-$C_{10}$-alkyl, $C_2$-$C_{10}$-alkenyl or $C_3$-$C_{10}$-alkynyl, in each case branched or unbranched, mono- or polysubstituted by F, Cl, Br, I, $NH_2$, SH or OH, $OCH_3$ or unsubstituted; $C_3$-$C_7$-cycloalkyl, saturated or unsaturated, mono- or polysubstituted by F, Cl, Br, I, $NH_2$, SH or OH, $OCH_3$ or unsubstituted, or a corresponding heterocyclic radical in which a C atom in the ring is replaced by S, O or $NR^3$,
where $R^3$ is chosen from
H, $C_1$-$C_{10}$-alkyl, $C_2$-$C_{10}$-alkenyl or $C_2$-$C_{10}$-alkynyl, in each case branched or unbranched, mono- or polysubstituted by F, Cl, Br, I, $NH_2$, SH or OH, $OCH_3$ or unsubstituted;
alkylaryl, saturated or unsaturated, mono- or polysubstituted by F, Cl, Br, I, $NH_2$, SH or OH, $OCH_3$ or unsubstituted;
alkylheteroaryl, saturated or unsaturated, mono- or polysubstituted by F, Cl, Br, I, $NH_2$, SH or OH, $OCH_3$ or unsubstituted, aryl, mono- or polysubstituted by F, Cl, Br, I, $NH_2$, SH or OH, $OCH_3$ or unsubstituted;

or

$R^1$ and $R^2$ together form a $C_3$-$C_7$-cycloalkyl, saturated or unsaturated, mono- or polysubstituted by F, Cl, Br, I, $NH_2$, SH or OH, $OCH_3$ or unsubstituted, or a corresponding heterocyclic radical in which a C atom in the ring is replaced by S, O or $NR^4$,
where $R^4$ is chosen from
H, $C_1$-$C_{10}$-alkyl, $C_2$-$C_{10}$-alkenyl or $C_3$-$C_{10}$-alkynyl, in each case branched or unbranched, mono- or polysubstituted by F, Cl, Br, I, $NH_2$, SH or OH, $OCH_3$ or unsubstituted,

and
G is chosen from pyridin-3-yl or pyridin-4-yl and is unsubstituted or mono- or polysubstituted, wherein at least one hydrogen radical is replaced by F, Cl, Br, I, CN, $CF_3$, $NO_2$, $C_1$-$C_{10}$-alkyl, $C_2$-$C_{10}$-alkenyl or $C_3$-$C_{10}$-alkynyl, in each

case branched or unbranched, mono- or polysubstituted by F, Cl, Br, I, $NH_2$, SH or OH, $OCH_3$ or unsubstituted; $C_3$-$C_7$-cycloalkyl, saturated or unsaturated, mono- or polysubstituted by F, Cl, Br, I, $NH_2$, SH or OH, $OCH_3$ or unsubstituted, or a corresponding heterocyclic radical in which a C atom in the ring is replaced by S, O or $NR^5$, where $R^5$ is chosen from
H, $C_1$-$C_{10}$-alkyl, $C_2$-$C_{10}$-alkenyl or $C_2$-$C_{10}$-alkynyl, in each case branched or unbranched, mono- or polysubstituted by F, Cl, Br, I, $NH_2$, SH or OH, $OCH_3$ or unsubstituted;
$OR^6$, $OC(O)R^6$, $OC(S)R^6$, $C(O)R^6$, $C(O)OR^6$, $C(S)_R{}^6$, $C(S)OR^6$, $SR^6$, $S(O)R^6$ or $S(O_2)R^6$, wherein $R^6$ is chosen from H, $C_1$-$C_{10}$-alkyl, $C_2$-$C_{10}$-alkenyl or $C_2$-$C_{10}$-alkynyl, in each case branched or unbranched, mono- or polysubstituted by F, Cl, Br, I, $NH_2$, SH or OH, $OCH_3$ or unsubstituted; $C_3$-$C_7$-cycloalkyl, saturated or unsaturated, mono- or polysubstituted by F, Cl, Br, I; $NH_2$, SH or OH, $OCH_3$ or unsubstituted, or a corresponding heterocyclic radical in which a C atom in the ring is replaced by S, O or $NR^7$, where $R^7$ is chosen from
H, $C_1$-$C_{10}$-alkyl, $C_2$-$C_{10}$-alkenyl or $C_3$-$C_{10}$-alkynyl, in each case branched or unbranched, mono- or polysubstituted by F, Cl, Br, I, $NH_2$, SH or OH, $OCH_3$ or unsubstituted;
alkylaryl, saturated or unsaturated, mono- or polysubstituted by F, Cl, Br, I, $NH_2$, SH or OH, $OCH_3$ or unsubstituted; aryl or heteroaryl, in each case mono- or polysubstituted by F, Cl, Br, I, $NH_2$, SH or OH, $OCH_3$ or unsubstituted;
$NR^8R^9C(O)NR^8R^9$ or $S(O_2)NR^8R^9$, wherein $R^8$ and $R^9$ independently of one another are chosen from
H, $C_1$-$C_{18}$-alkyl, $C_2$-$C_{18}$-alkenyl or $C_3$-$C_{18}$-alkynyl, in each case branched or unbranched, mono- or polysubstituted by F, Cl, Br, I, $NH_2$, SH or OH, $OCH_3$ or unsubstituted; $C_3$-$C_7$-cycloalkyl, saturated or unsaturated, mono- or polysubstituted by F, Cl, Br, I, $NH_2$, SH or OH, $OCH_3$ or unsubstituted, or a corresponding heterocyclic radical in which a C atom in the ring is replaced by S, O or $NR^{10}$, where $R^{10}$ is chosen from
H, $C_1$-$C_{10}$-alkyl, $C_2$-$C_{10}$-alkenyl or $C_3$-$C_{10}$-alkynyl, in each case branched or unbranched, mono- or polysubstituted by F, Cl, Br, I, $NH_2$, SH or OH, $OCH_3$ or unsubstituted;
alkylaryl, saturated or unsaturated, mono- or polysubstituted by F, Cl, Br, I, $NH_2$, SH or OH, $OCH_3$ or unsubstituted; aryl or heteroaryl, in each case mono- or polysubstituted by F, Cl, Br, I, $NH_2$, SH or OH, $OCH_3$ or unsubstituted;
or
$R^8$ and $R^9$ together form a $C_3$-$C_7$-cycloalkyl, saturated or unsaturated, mono- or polysubstituted by F, Cl, Br, I, $NH_2$, SH or OH, $OCH_3$ or unsubstituted, or a corresponding heterocyclic radical in which a C atom in the ring is replaced by S, O or $NR^{10}$, where $R^{10}$ is chosen from
H, $C_1$-$C_{10}$-alkyl, $C_2$-$C_{10}$-alkenyl or $C_3$-$C_{10}$-alkynyl, in each case branched or unbranched, mono- or polysubstituted by F, Cl, Br, I, $NH_2$, SH or OH, $OCH_3$ or unsubstituted;
alkylaryl, aryl or heteroaryl, in each case mono- or polysubstituted by F, Cl, Br, I, $NH_2$, SH or OH, $OCH_3$ or unsubstituted,
also in the form of their racemates, enantiomers and diastereomers, in particular mixtures of their enantiomers or diastereomers or an individual enantiomer or diastereomer and their free bases or a salt formed with a physiologically acceptable acid, in particular the hydrochloride salt.

**2.** Compounds of the general formula I according to claim 1, **characterized in that** W is $CH_2$ and n = 0 - 3.

**3.** Compounds of the general formula I according to claim 1, **characterized in that** W is O, S, SO or $SO_2$ and n = 0 - 3

**4.** Compounds of the general formula I according to claim 3, **characterized in that** W is O or S and n = 0 - 3.

**5.** Compounds of the general formula I according to one of claims 1 to 4, **characterized in that**

$R_1$ and $R_2$ independently of one another are chosen from H, $C_1$-$C_{10}$-alkyl, alkylaryl, saturated or unsaturated, mono- or polysubstituted by F, Cl, Br, I, $NH_2$, SH or OH, $OCH_3$ or unsubstituted; alkylheteroaryl, saturated or unsaturated, mono or polysubstituted by F, Cl, Br, I, $NH_2$, SH or OH, $OCH_3$ or unsubstituted,

or

$R_1$ and $R_2$ together form a $C_3$-$C_7$-cycloalkyl, saturated or unsaturated, mono- or polysubstituted by F, Cl, Br, I, $NH_2$, SH or OH, $OCH_3$ or unsubstituted,

and the radical G is unsubstituted or substituted by Cl, Br or $OR_6$, wherein $R_6$ is H or $C_1$-$C_{10}$-alkyl.

**6.** Compounds of the general formula I according to one of claims 1, 2 and 5, wherein the compounds are chosen from:

dimethyl-(2-pyridin-3-yl-cyclohex-1-enylmethyl)-amine and the corresponding dihydrochloride **(1)**
methyl-(2-pyridin-3-yl-cyclohex-l-enylmethyl)-amine and the corresponding hydrochloride **(2)**
dimethyl-(2-pyridin-3-yl-cyclohex-2-enylmethyl)amine and the corresponding hydrochloride **(4)**
dimethyl-(2-pyridin-3-yl-cyclohexylmethyl)-amine and the corresponding hydrochloride; diastereoisomer 1 **(6)**
dimethyl-(2-pyridin-3-yl-cyclohexylmethyl)-amine and the corresponding hydrochloride; diastereoisomer 2 **(7)**
benzyl-methyl-(2-pyridin-3-yl-cyclohex-1-enylmethyl)-amine and the corresponding hydrochloride (10)
benzyl-methyl-(2-pyridin-3-yl-cyclohex-2-enylmethyl)-amine and the corresponding hydrochloride **(11)**
[2-chloro-2-(6-chloro-pyridin-3-yl)-cycloheptylmethyl]-dimethyl-amine and the corresponding hydrochloride (13)
(2-chloro-2-pyridin-3-yl-cyclohexylmethyl)-dimethylamine and the corresponding hydrochloride **(14)**
[2-chloro-2-(6-chloro-pyridin-3-yl)-cyclohexylmethyl]-dimethylamine and the corresponding hydrochloride **(15)**
mixture of [2-(4-bromo-6-chloro-pyridin-3-yl)-cyclohex-1-enylmethyl]-methyl-amine and [2-(4-bromo-6-chloro-pyridin-3-yl)-cyclohex-2-enylmethyl]-methyl-amine and the corresponding hydrochlorides **(20)**
dimethyl-(2-pyridin-3-yl-cyclohept-l-enylmethyl)-amine and the corresponding hydrochloride **(23)**
dimethyl-(2-pyridin-3-yl-cyclohept-2-enylmethyl)-amine and the corresponding hydrochloride **(24)**
mixture of [2-(6-chloro-pyridin,-3-yl)-cyclooct-1-enylmethyl]-dimethyl-amine hydrochloride and [2-(6-chloro-pyridin-3-yl)-cyclooct-2-enylmethyl]-dimethylamine hydrochloride **(25)**
dimethyl-(2-pyridin-3-yl-cycloheptylmethyl)-amine and the corresponding hydrochloride; diastereoisomer 1 **(26)**
dimethyl-(2-pyridin-3-yl-cycloheptylmethyl)-amine and the corresponding hydrochloride; diastereoisomer 2 **(27)**
dimethyl-(2-pyridin-3-yl-cyclopent-1-enylmethyl)-amine and the corresponding hydrochloride **(28)**
dimethyl-(2-pyridin-3-yl-cyclopent-2-enylmethyl)-amine and the corresponding hydrochloride **(29)**
dimethyl-(2-pyridin-3-yl-cyclopentylmethyl)-amine and the corresponding hydrochloride **(31)**
dimethyl-(2-pyridin-3-yl-cyclooct-1-enylmethyl)-amine and the corresponding hydrochloride **(32)**
dimethyl-(2-pyridin-3-yl-cyclooct-2-enylmethyl)-amine and the corresponding hydrochloride **(33)**
dimethyl-(2-pyridin-3-yl-cyclooctylmethyl)-amine and the corresponding hydrochloride **(35)**
(2-chloro-2-pyridin-4-yl-cycloheptylmethyl)-dimethylamine and the corresponding hydrochloride **(36)**
dimethyl-(2-pyridin-4-yl-cyclohept-2-enylmethyl)-amine and the corresponding hydrochloride **(37)**
dimethyl-(2-pyridin-4-yl-cyclohept-1-enylmethyl)-amine and the, corresponding hydrochloride **(38)**
dimethyl-(2-pyridin-4-yl-cyclohex-1-enylmethyl)-amine and the corresponding hydrochloride **(39)**
mixture of dimethyl-(2-pyridin-4-yl-cyclohex-1-enylmethyl)-amine hydrochloride and dimethyl-(2-pyridin-4-yl-cyclohex-2-enylmethyl)-amine hydrochloride **(40)**
dimethyl-(2-pyridin-4-yl-cyclooct-1-enylmethyl)-amine and the corresponding hydrochloride **(42)**
dimethyl-(2-pyridin-4-yl-cyclopent-2-enylmethyl)-amine and the corresponding hydrochloride **(43)**
dimethyl-(2-pyridin-4-yl-cyclopent-1-enylmethyl)-amine and the corresponding hydrochloride **(47)**
[2-(6-chloro-pyridin-3-yl)-cyclohept-2-enylmethyl]-dimethyl-amine and the corresponding hydrochloride **(48)**
[2-(6-chloro-pyridin-3-yl)-cyclohex-1-enylmethyl]-dimethyl-amine and the corresponding hydrochloride **(49)**
[2-(5-methoxy-pyridin-3-yl)-cyclohex-1-enylmethyl]-dimethyl-amine and the corresponding hydrochloride **(54)**
[2-(5-methoxy-pyridin-3-yl)-cyclohex-2-enylmethyl]-dimethyl-amine and the corresponding hydrochloride **(55)**
mixture of [2-(5-methoxy-pyridin-3-yl)-cyclohept-1-enylmethyl]-dimethyl-amine hydrochloride and [2-(5-methoxy-pyridin-3-yl)-cyclohept-2-enylmethyl]-dimethylamine hydrochloride **(58)**
mixture of 5-(2-dimethylaminomethyl-cyclohept-1-enyl)-pyridin-3-ol; hydrochloride and 5-(7-dimethylaminomethyl-cyclohept-1-enyl)-pyridin-3-ol; hydrochloride **(59)**
[2-(5-methoxy-pyridin-3-yl)-cycloheptylmethyl]-dimethyl-amine and the corresponding hydrochloride **(60)**
[2-(5-methoxy-pyridin-3-yl)-cyclohexylmethyl]-dimethylamine and the corresponding hydrochloride **(62)**
mixture of [2-(5-methoxy-pyridin-3-yl)-cyclohexylmethyl]-dimethyl-amine (diastereoisomer 1) and [2-(5-methoxy-pyridin-3-yl)-cyclohexylmethyl]-dimethyl-amine (diastereoisomer 2) and the corresponding hydrochlorides **(63)**
5-(2-dimethylaminomethyl-cyclohex-1-enyl)-pyridin-3-ol and the corresponding hydrochloride **(64)**
dipropyl-(2-pyridin-3-yl-cyclohex-1-enylmethyl)-amine and the corresponding hydrochloride **(65)**
dipropyl-(2-pyridin-3-yl-cyclohex-2-enylmethyl)-amine and the corresponding hydrochloride **(66)**
[2-(5-methoxy-pyridin-3-yl)-cyclooct-1-enylmethyl]-dimethyl-amine hydrochloride and [2-(5-methoxy-pyridin-3-yl)-cyclooct-2-enylmethyl]-dimethyl-amine and the corresponding hydrochlorides **(67)**
[2-(6-chloro-pyridin-3-yl)-cyclohex-1-enylmethyl]-dimethyl-amine and the corresponding hydrochloride **(68)**
[2-(6-chloro-pyridin-3-yl)-cyclohex-2-enylmethyl]-dimethyl-amine and the corresponding hydrochloride **(69)**
[2-(5-methoxy-pyridin-3-yl)-cyclooctylmethyl]-dimethylamine and the corresponding hydrochloride; diastereoisomer 1 **(70)**
[2-(6-chloro-pyridin-3-yl)-cyclooct-2-enylmethyl]-dimethyl-amine and the corresponding hydrochloride **(71)**
[2-(5-methoxy-pyridin-3-yl)-cyclooctylinethyl]-dimethylamine and the corresponding hydrochloride; diastereoisomer 2 **(72)**

[2-(5-methoxy-pyridin-3-yl)-cyclooct-2-enylmethyl]-dimethyl-amine and the corresponding hydrochloride **(73)**
[2-(5-methoxy-pyridin-3-yl)-cyclooct-l-enylmethyl]-dimethyl-amine and the corresponding hydrochloride **(74)**
5-(7-dimethylaminomethyl-cyclohept-l-enyl)-pyridin-3-ol and the corresponding hydrochloride **(75)**
5-(2-dimethylaminomethyl-cyclohept-l-enyl)-pyridin-3-ol and the corresponding hydrochloride **(76)**
5-(2-dimethylaminomethyl-cycloheptyl)-pyridin-3-ol and the corresponding hydrochloride **(77)**
methyl-(2-pyridin-3-yl-cyclohex-2-enylmethyl)-amine and the corresponding hydrochloride **(79)**.

**7.** Compound of the general formula **I** according to one of claims 1, 3, 4 and 5, wherein the compounds are chosen from:

3-dimethylaminomethyl-4-pyridin-3-yl-tetrahydrothiopyran-4-ol and the corresponding hydrochloride; diastereoisomer 1 **(50)**
3-dimethylaminomethyl-4-pyridin-3-yl-tetrahydrothiopyran-4-ol and the corresponding hydrochloride; diastereoisomer 2 **(51)**
3-dimethylaminomethyl-4-pyridin-3-yl-tetrahydro-pyran-4-ol and the corresponding hydrochloride **(52)**
dimethyl-(4-pyridin-3-yl-5,6-dihydro-2H-pyran-3-yl-methyl)-amine and the corresponding hydrochloride **(53)**.

**8.** Process for the preparation of compounds of the general formula **I** and their pharmaceutically acceptable salts, **characterized in that** cycloalkanones of the general formula **II** are reacted with immonium salts of the formula **III** or with paraformaldehyde and an amine of the formula **IV**, wherein $R^{10}$ has a meaning analogous to $R^1$ and $R^{11}$ has a meaning analogous to $R^2$, the Mannich bases obtained are reacted with an organometallic compound of the formula **V,** in which Z denotes lithium and V has a meaning corresponding to G, in a solvent at between -70 °C and 60 °C, wherein the organolithium compound of the formula **V**, in which Z denotes Li and V has a meaning corresponding to G, is prepared by halogen-lithium exchange by reaction of halogen compounds of the formula **VI**, in which A denotes Cl, Br or I and V has a meaning corresponding to G, with preferably n-butyllithium solution, as a result of which products of the general formula **VII,** in which $R^{10}$ has a meaning analogous to $R^1$, $R^{11}$ has a meaning analogous to $R^2$ and V has a meaning corresponding to G, are obtained, and these are reacted with thionyl chloride and the products are subsequently worked up under basic conditions to give a mixture of compounds of the general formulae **VIII, IXa** and **IXb,** in which $R^{10}$ has a meaning analogous to $R^1$, $R^{11}$ has a meaning analogous to $R^2$ and V has a meaning corresponding G, the compounds are separated and hydrogenation of the compounds of the general formulae **IXa** and **IXb** is subsequently carried out with catalytically activated hydrogen, platinum or palladium absorbed on a support material serving as the catalyst, in a solvent under pressures of 0.1 to 10 bar and at temperatures of 20 °C to 80 °C to give compounds of the formula **X**, in which $R^{10}$ has a meaning analogous to $R^1$, $R^{11}$ has a meaning analogous to $R^2$, and V has a meaning corresponding to G.

**9.** Process according to claim 8, **characterized in that** at least one OH group contained in formula **I** is replaced by ah $OSi(Ph)_2$tert-but group, at least one SH group is replaced by an S-p-methoxybenzyl group and/or at least one $NH_2$ group is replaced by an $NO_2$ group and, after the entire reaction sequence, an $OSi(Ph)_2$tert-but group is split off with tetrabutylammonium fluoride in tetrahydrofuran and/or at least one p-methoxybenzyl group is split off with a metal amine, preferably sodium amine, and/or at least one $NO_2$ group is reduced to $NH_2$.

**10.** Process according to claims 8 and 9, **characterized in that** after the BuLi reaction, a process product having at least one $C(O)OCH_3$ and/or $C(S)OCH_3$ group is hydrolysed with KOH solution or NaOH solution in methanol at 40 °C - 60 °C.

**11.** Medicaments comprising at least one compound of the general formula **I** or one of its pharmaceutically acceptable salts.

**12.** Use of a compound of the general formula **I** or of one of its pharmaceutically acceptable salts for the preparation of a medicament for'treatment of depressions, anxiety disorders and/or pain.

**13.** Pharmaceutical composition which comprises at least one compound of the general formula **I** or one of its pharmaceutically acceptable salts and at least one pharmaceutical auxiliary substance.

## Revendications

**1.** Hétéroarylcycloalkylméthylamines saturées et non saturées de formule générale I

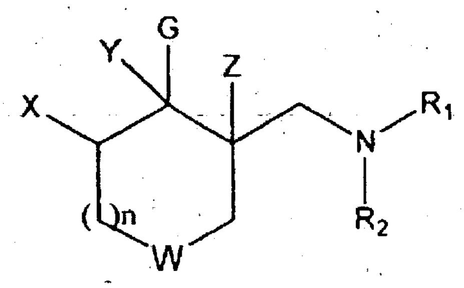

formule dans laquelle

W représente $CH_2$, Y étant alors choisi entre H ou Cl en même temps que X et Z représentent H,
ou bien
X et Y forment ensemble une liaison et Z représente H,
ou bien
Y et Z forment ensemble une liaison et X représente H,

ou bien

W représente 0, S, SO ou $SO_2$, Y étant choisi entre H, OH, Cl en même temps que X et Z représentent H
ou bien
X et Y forment ensemble une liaison et Z représente H,
ou bien
Y et Z forment ensemble une liaison et X représente H,

et n a une valeur de 0 à 3,

$R^1$ et $R^2$ sont choisis indépendamment l'un de l'autre entre H, un reste alkyle en $C_1$ à $C_{10}$, alcényle en $C_2$ à $C_{10}$ ou alcynyle en $C_3$ à $C_{10}$, chacun ramifié ou non ramifié, substitué une ou plusieurs fois par F, Cl, Br, I, $NH_2$, SH ou OH, $OCH_3$, ou non substitué ; un reste cycloalkyle en $C_3$ à $C_7$, saturé ou non saturé, substitué une ou plusieurs fois par F, Cl, Br, I, $NH_2$, SH ou OH, $OCH_3$, ou non substitué, ou un hétérocycle correspondant dans lequel un atome de carbone du noyau est remplacé par S, O ou $NR^3$,
$R^3$ étant choisi entre
H, un reste alkyle en $C_1$ à $C_{10}$, alcényle en $C_2$ à $C_{10}$ ou alcynyle en $C_2$ à $C_{10}$, chacun non ramifié ou ramifié, substitué une ou plusieurs fois par F, Cl, Br, I, $NH_2$, SH ou OH, $OCH_3$, ou non substitué ;
un reste alkylaryle, saturé ou non saturé, substitué une ou plusieurs fois par F, Cl, Br, I, $NH_2$, SH ou OH, $OCH_3$, ou non substitué ; un reste alkylhétéroaryle, saturé ou non saturé, substitué une ou plusieurs fois par F, Cl, Br, I, $NH_2$, SH ou OH, $OCH_3$, ou non substitué, un reste aryle, substitué une ou plusieurs fois par F, Cl, Br, I, $NH_2$, SH ou OH, $OCH_3$, ou non substitué ;

ou bien

$R^1$ et $R^2$ forment ensemble un reste cycloalkyle en $C_3$ à $C_7$, saturé ou non saturé, substitué une ou plusieurs fois par F, Cl, Br, I, $NH_2$, SH ou OH, $OCH_3$, ou non substitué, ou un hétérocycle correspondant dans lequel un atome de carbone du noyau est remplacé par S, O ou $NR^4$,
$R^4$ étant choisi entre
H, un reste alkyle en $C_1$ à $C_{10}$, alcényle en $C_2$ à $C_{10}$ ou alcynyle en $C_3$ à $C_{10}$, chacun ramifié ou non ramifié, substitué une ou plusieurs fois par F, Cl, Br, I, $NH_2$, SH ou OH, $OCH_3$, ou non substitué,

et
G est choisi entre un groupe pyridine-3-yle ou pyridine-4-yle et est non substitué ou substitué une ou plusieurs fois, au moins un reste hydrogène étant remplacé par F, Cl, Br, I, CN, $CF_3$, $NO_2$, un radical alkyle en $C_1$ à $C_{10}$, alcényle en $C_2$ à $C_{10}$ ou alcynyle en $C_3$ à $C_{10}$, chacun ramifié ou non ramifié, substitué une ou plusieurs fois par F, Cl, Br, I, $NH_2$, SH ou OH, $OCH_3$, ou non substitué ; un reste cycloalkyle en $C_3$ à $C_7$, saturé ou non saturé, substitué une ou plusieurs fois par F, Cl, Br, I, $NH_2$, SH ou OH, $OCH_3$ ou non substitué, ou un hétérocycle correspondant dans lequel un atome de carbone du noyau est remplacé par S, O ou $NR^5$, $R^5$ étant choisi entre

H, un radical alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH, $OCH_3$, ou non substitué ;
un groupe $OR^6$, $OC(O)R^6$, $OC(S)R^6$, $C(O)R^6$, $C(O)OR^6$, $C(S)R^6$, $C(S)OR^6$, $SR^6$, $S(O)R^6$ ou $S(O_2)R^6$, $R^6$ étant choisi entre
H, un radical alkyle en $C_1$ à $C_{10}$, alcényle en $C_2$ à $C_{10}$ ou alcynyle en $C_2$ à $C_{10}$, chacun ramifié ou non ramifié, substitué une ou plusieurs fois par F, Cl, Br, I, $NH_2$, SH ou OH, $OCH_3$, ou non substitué ; un reste cycloalkyle en $C_3$ à $C_7$, saturé ou non saturé, substitué une ou plusieurs fois par F, Cl, Br, I, $NH_2$, SH ou OH, $OCH_3$, ou non substitué ; ou un hétérocycle correspondant dans lequel un atome de carbone du noyau est remplacé par S, O ou $NR^7$, $R^7$ étant choisi entre
H, un radical alkyle en $C_1$ à $C_{10}$, alcényle en $C_2$ à $C_{10}$ ou alcynyle en $C_3$ à $C_{10}$, chacun ramifié ou non ramifié, substitué une ou plusieurs fois par F, Cl, Br, I, $NH_2$, SH ou OH, $OCH_3$, ou non substitué ;
un reste alkylaryle, saturé ou non saturé, substitué une ou plusieurs fois par F, Cl, Br, I, $NH_2$, SH ou OH, $OCH_3$, ou non substitué ; un reste aryle ou hétéroaryle, chacun substitué une ou plusieurs fois par F, Cl, Br, I, $NH_2$, SH ou OH, $OCH_3$, ou non substitué ;
un groupe $NR^8R^9C(O)NR^8R^9$ ou $S(O_2)NR^8R^9$, où $R^8$ et $R^9$ sont choisis indépendamment l'un de l'autre entre
H, un reste alkyle en $C_1$ à $C_{18}$, alcényle en $C_2$ à $C_{18}$ ou alcynyle en $C_3$ à $C_{18}$, chacun ramifié ou non ramifié, substitué une ou plusieurs fois par F, Cl, Br, I, $NH_2$, SH ou OH, $OCH_3$, ou non substitué ; un reste cycloalkyle en $C_3$ à $C_7$, saturé ou non saturé, substitué une ou plusieurs fois par F, Cl, Br, I, $NH_2$, SH ou OH, $OCH_3$, ou non substitué, ou un hétérocycle correspondant dans lequel un atome de carbone du noyau est remplacé par S, O ou $NR^{10}$, $R^{10}$ étant choisi entre
H, un radical alkyle en $C_1$ à $C_{10}$, alcényle en $C_2$ à $C_{10}$ ou alcynyle en $C_3$ à $C_{10}$, chacun ramifié ou non ramifié, substitué une ou plusieurs fois par F, Cl, Br, I, $NH_2$, SH ou OH, $OCH_3$, ou non substitué ;
un reste alkylaryle, saturé ou non saturé, substitué une ou plusieurs fois par F, Cl, Br, I, $NH_2$, SH ou OH, $OCH_3$, ou non substitué ; un reste aryle ou hétéroaryle, chacun substitué une ou plusieurs fois par F, Cl, Br, I, $NH_2$, SH ou OH, $OCH_3$, ou non substitué ;
ou bien
$R^8$ et $R^9$ forment ensemble un reste cycloalkyle en $C_3$ à $C_7$, saturé ou non saturé, substitué une ou plusieurs fois par F, Cl, Br, I, $NH_2$, SH ou OH, $OCH_3$, ou non substitué, ou un hétérocycle correspondant dans lequel un atome de carbone du noyau est remplacé par S, O ou $NR^{10}$, $R^{10}$ étant choisi entre
H, un radical alkyle en $C_1$ à $C_{10}$, alcényle en $C_2$ à $C_{10}$ ou alcynyle en $C_3$ à $C_{10}$, chacun ramifié ou non ramifié, substitué une ou plusieurs fois par F, Cl, Br, I, $NH_2$, SH ou OH, $OCH_3$, ou non substitué ;
un reste alkylaryle, aryle ou hétéroaryle, chacun substitué une ou plusieurs fois par F, Cl, Br, I, $NH_2$, SH ou OH, $OCH_3$, ou non substitué,
également sous forme de leurs racémates ; leurs énantiomères, leurs diastéréo-isomères, en particulier sous forme de mélanges de leurs énantiomères ou de leurs diastéréo-isomères ou d'un énantiomère ou d'un diastéréo-isomère individuel ainsi que de leurs bases libres ou d'un sel formé avec un acide physiologiquement acceptable, en particulier le chlorhydrate.

**2.** Composés de formule générale I suivant la revendication 1, **caractérisés en ce que** W représente $CH_2$ et n a une valeur de 0 à 3.

**3.** Composés de formule générale I suivant la revendication 1, **caractérisés en ce que** W représente O, S, SO ou $SO_2$ et n a une valeur de 0 à 3.

**4.** Composés de formule générale I suivant la revendication 3, **caractérisés en ce que** W représente O ou S et n a une valeur de 0 à 3.

**5.** Composés de formule générale I suivant l'une des revendications 1 à 4, **caractérisés en ce que**

$R^1$ et $R^2$ sont choisis indépendamment l'un de l'autre entre H, un reste alkyle en $C_1$ à $C_{10}$, un reste alkylaryle, saturé ou non saturé, substitué une ou plusieurs fois par F, Cl, Br, I, $NH_2$, SH ou OH, $OCH_3$, ou non substitué ; un reste alkylhétéroaryle, saturé ou non saturé, substitué une ou plusieurs fois par F, Cl, Br, I, $NH_2$, SH ou OH, $OCH_3$, ou non substitué,

ou bien

$R^1$ et $R_2$ forment ensemble un reste cycloalkyle en $C_3$ à $C_7$, saturé ou non saturé, substitué une ou plusieurs fois par F, Cl, Br, I, $NH_2$, SH ou OH, $OCH_3$, ou non substitué,

et le reste G est non substitué ou substitué par Cl, Br ou $OR_6$, $R_6$ représentant H ou un radical alkyle en $C_1$ à $C_{10}$.

**6.** Composés de formule générale I suivant l'une des revendications 1, 2 et 5, ces composés étant choisis entre :

Diméthyl-(2-pyridine-3-yl-cyclohex-1-énylméthyl)-amine et le dichlorhydrate correspondant (1)
Méthyl-(2-pyridine-3-yl-cyclohéx-1-énylméthyl)-amine et le chlorhydrate correspondant (2)
Diméthyl-(2-pyridine-3-yl-cyclohex-2-énylméthyl)-amine et le chlorhydrate correspondant (4)
Diméthyl-(2-pyridine-3-yl-cyclohexylméthyl)-amine et le chlorhydrate correspondant ; diastéréo-isomère 1 (6)
Diméthyl-(2-pyridine-3-yl-cyclohexylméthyl)-amine et le chlorhydrate correspondant ; diastéréb-isomère 2 (7)
Benzyl-méthyl-(2-pyridine-3-yl-cyclohex-1-énylméthyl)-amine et le chlorhydrate correspondant (10)
Benzyl-méthyl-(2-pyridine-3-yl-cyclohex-2-énylméthyl)-amine et le chlorhydrate correspondant (11)
[2-chloro-2-(6-chloropyridine-3-yl)-cycloheptylméthyl]-diméthylamine et le chlorhydrate correspondant (13)
(2-chloro-2-pyridine-3-yl-cyclohexylméthyl)-diméthylamine et le chlorhydrate correspondant (14)
[2-chloro-2'-(6-chloropyridine-3-yl)-cyclohexylméthyl]-diméthylamine et le chlorhydrate correspondant (15)
Mélange de [2-(4-bromo-6-chloropyridine-3-yl)-cyclohex-1-énylméthyl]-méthylamine et de [2-(4-bromo-6-chloropyridine-3-yl)-cyclohex-2-énylméthyl]-méthylamine et les chlorhydrates correspondants (20)
Diméthyl-(2-pyridine-3-yl-cyclohept-1-énylméthyl)-amine et le chlorhydrate correspondant (23)
Diméthyl-(2-pyridine-3-yl-cyclohept-2-énylméthyl)-amine et le chlorhydrate correspondant (24)
Mélange de chlorhydrate de [2-(6-chloropyridine-3-yl)-cyclo-oct-1-énylméthyl]-diméthylamino et de chlorhydrate de [2-(6-chloropyridine-3-yl)-cyclo-oct-2-énylméthyl]-diméthylamine (25)
Diméthyl-(2-pyridine-3-yl-cycloheptylméthyl)-amine et le chlorhydrate correspondant ; diastéréo-isomère 1 (26)
Diméthyl-(2-pyridine-3-yl-cycloheptylméthyl)-amine et le chlorhydrate correspondant ; diastéréo-isomère 2 (27)
Diméthyl-(2-pyridine-3-yl-cyclopent-1-énylméthyl)-amine et le chlorhydrate correspondant (28)
Diméthyl-(2-pyridine-3-yl-cyclopent-2-énylméthyl)-amine et le chlorhydrate correspondant (29)
Diméthyl-(2-pyridine-3-yl-cyclopentylméthyl)-amine et le chlorhydrate correspondant (31)
Diméthyl-(2-pyridine-3-yl-cyclo-oct-1-énylméthyl)-amine et le chlorhydrate correspondant (32)
Diméthyl-(2-pyridine-3-yl-cyclo-oct-2-énylméthyl)-amine et le chlorhydrate correspondant (33)
Diméthyl-(2-pyridine-3-yl-cyclo-octylméthyl)-amine et le chlorhydrate correspondant (35)
(2-chloro-2-pyridine-4-yl-cycloheptylméthyl)-diméthylamine et le chlorhydrate correspondant (36)
Diméthyl-(2-pyridine-4-yl-cyclohept-2-énylméthyl)-amine et le chlorhydrate correspondant (37)
Diméthyl-(2-pyridine-4-yl-cyclohept-1-énylméthyl)-amine et le chlorhydrate correspondant (38)
Diméthyl-(2-pyridine-4-yl-cyclohex-1-énylméthyl)-amine et le chlorhydrate correspondant (39)
Mélange de chlorhydrate de diméthyl-(2-pyridine-4-yl-cyclohex-1-énylméthyl)amine et de chlorhydrate de diméthyl-(2-pyridine-4-yl-cyclohex-2-énylméthyl)-amine (40)
Diméthyl-(2-pyridine-4-yl-cyclo-oct-1-énylméthyl)-amine et le chlorhydrate correspondant (42)
Diméthyl-(2-pyridine-4-yl-cyclopent-2-énylméthyl)-amine et le chlorhydrate correspondant (43)
Diméthyl-(2-pyridine-4-yl-cyclopent-1-énylméthyl)-amine et le chlorhydrate correspondant (47)
[2-(6-chloropyridine-3-yl)-cyclohept-2-énylméthyl]-diméthylamine et le chlorhydrate correspondant (48)
[2-(6-chloropyridine-3-yl)-cyclohex-1-énylméthyl]-diméthylamine et le chlorhydrate correspondant (49)
[2-(5-méthoxypyridine-3-yl)-cyclohex-1-énylméthyl]-diméthylamine et le chlorhydrate correspondant (54)
[2-(5-méthoxypyridine-3-yl)-cyclohex-2-énylméthyl]-diméthylamine et le chlorhydrate correspondant (55)
Mélange de chlorhydrate de [2-(5-méthoxypyridine-3-yl)-cyclohept-1-énylméthyl]-diméthylamine et de chlorhydrate de [2-(5-méthoxypyridine-3-yl)-cyclohept-2-énylméthyl]-diméthylamine (58)
Mélange de chlorhydrate de 5-(2-diméthylaminométhylcyclohept-1-ényl)-pyridine-3-ol et de chlorhydrate de 5-(7-diméthylaminométhyl-cyclohept-1-ényl)-pyridine-3-ol (59)
[2-(5-méthoxypyridine-3-yl)-cycloheptylméthyl]-diméthylamine et le chlorhydrate correspondant (60)
[2-(5-méthoxypyridine-3-yl)-cyclohexylméthyl]-diméthylamine et le chlorhydrate correspondant (62)
Mélange de 2-(5-méthoxypyridine-3-yl)-cycloliexylméthyl]-diméthylamine (diastéréo-isomère 1) et de [2-(5-méthoxypyridine-3-yl)-cyclohexylméthyl]-diméthylamine (diastéréo-isomère 2) et des chlorhydrates correspondants (63)
5-(2-diméthylaminométhyl-cyclohex-1-ényl)-pyridine-3-ol et le chlorhydrate correspondant (64)
Dipropyl-(2-pyridine-3-yl-cyclohex-1-énylméthyl)-amine et le chlorhydrate correspondant (65)
Dipropyl-(2-pyridine-3-yl-cyclohex-2-énylméthyl)-amine et le chlorhydrate correspondant (66)
Chlorhydrate de [2-(5-méthoxypyridine-3-yl)-cyclo-oct-1-énylméthyl]-diméthylamine et [2-(5-méthoxypyridine-3-yl)-cyclo-oct-2-énylméthyl]-diméthylamine et les chlorhydrates correspondants (67)
[2-(6-chloropyridine-3-yl)-cyclohex-1-énylméthyl]-diméthylamine et le chlorhydrate correspondant (68)
[2-(6-chloropyridine-3-yl)-cyclohex-2-énylméthyl]-diméthylamine et le chlorhydrate correspondant (69)
[2-(5-méthoxypyridine-3-yl)-cyclo-octylméthyl]-diméthylamine et le chlorhydrate correspondant ; diastéréo-isomère 1 (70)
[2-(6-chloropyridine-3-yl)-cyclo-oct-2-énylméthyl]-diméthylamine et le chlorhydrate correspondant (71)
[2-(5-méthoxypyridine-3-yl)-cyclo-octylméthyl]-diméthylamine et le chlorhydrate correspondant ; diastéréo-isomère 2 (72)
[2-(5-méthoxypyridine-3-yl)-cyclo-oct-2-énylméthyl]-diméthylamine et le chlorhydrate correspondant (73)
[2-(5-méthoxypyridine-3-yl)-cyclo-oct-1-énylméthyl]-diméthylamine et le chlorhydrate correspondant (74)
5-(7-diméthylaminométhyl-cyclohept-1-ényl)-pyridine-3-ol et le chlorhydrate correspondant (75)
5-(2-diméthylaminométhyl-cyclohept-1-ényl)-pyridine-3-ol et le chlorhydrate correspondant (76)
5-(2-diméthylaminométhyl-cycloheptyl)-pyridine-3-ol et le chlorhydrate correspondant (77)
Méthyl-(2-pyridine-3-yl-cyclohex-2-énylméthyl)-amine et le chlorhydrate correspondant (79).

**7.** Composé de formule générale I suivant l'une des revendications 1, 3, 4 et 5, les composés étant choisis entré :

3-diméthylaminométhyl-4-pyridine-3-yl-tétrahydrothiopyranne-4-ol et le chlorhydrate correspondant ; diastéréo-isomère 1 (50)
3-diméthylaminométhyl-4-pyridine-3-yl-tétrahydrothiopyranne-4-ol et le chlorhydrate correspondant ; diastéréo-isomère 2 (51)
3-diméthylaminométhyl-4-pyridine-3-yl-tétrahydropyranne-4-ol et le chlorhydrate correspondant (52)
Diméthyl-(4-pyridine-3-yl-5,6-dihydro-2H-pyranne-3-yl-méthyl)-amine et le chlorhydrate correspondant (53).

**8.** Procédé de production de composés de formule générale I ainsi que de leurs sels pharmaceutiquement acceptables, **caractérisé en ce que** des cycloalcanones de formule générale II sont amenées à réagir avec des sels d'immonium de formule III ou avec le paraformaldéhyde et une amine de formule IV, $R^{10}$ ayant une définition analogue à celle de $R^1$, et $R^{11}$ ayant une définition analogue à celle de $R^2$, les bases de Mannich obtenues sont amenées à réagir avec un composé organométallique de formule V, dans laquelle Z représente Li et V a une définition correspondant à G, dans des solvants entre - 70°C et 60°C, le composé organique de lithium de formule V, dans laquelle Z représente Li et V a une définition correspondant à G, étant préparé par réaction de composés halogénés de formule VI, dans laquelle A représente Cl, Br ou I et V a une définition correspondant à G, avec avantageusement une solution de n-butyllithium dans l'hexane par échange halogène-lithium, ce qui permet d'obtenir des produits de formule générale VII dans laquelle $R^{10}$ a une définition analogue à celle de $R^1$, $R^{11}$ a une définition analogue à celle de $R^2$ et V a une définition correspondant à G, qu'on fait réagir avec le chlorure de thionyle et qu'on traite ensuite dans des conditions basiques pour obtenir un mélange de composés de formules générales VIII, IXa et IXb dans lesquelles $R^{10}$ a une définition analogue à celle de $R^1$, $R^{11}$ a une définition analogue à celle de $R^2$ et V a une définition correspondant à G, on sépare les composés, on effectue ensuite l'hydrogénation des composés de formules générales IXa et IXb avec de l'hydrogène catalytiquement activé, en utilisant comme catalyseur le platine ou le palladium absorbé sur une matière de support, dans des solvants à des pressions de 0,1 à 10 bars et à des températures de 20°C à 80°C pour obtenir des composés de formule X dans laquelle $R^{10}$ a une définition analogue à celle de $R^1$, $R^{11}$ a une définition analogue à celle de $R^2$ et V a une définition correspondant à G.

**9.** Procédé suivant la revendication 8, **caractérisé en ce qu'**au moins un groupe OH contenu dans la formule I est remplacé par un groupe $OSi(Ph)_2$tertiobutyle, au moins un groupe SH est remplacé par un groupe S-p-méthoxybenzyle et/ou au moins un groupe $NH_2$ est remplacé par un groupe $NO_2$ et, après la fin de la séquence réactionnelle totale, un groupe $OSi(Ph)_2$tertiobutyle est éliminé avec le fluorure de tétrabutylammonium dans le tétrahydrofuranne et/ou au moins un groupe p-méthoxybenzyle est éliminé avec un dérivé métallique d'amine, avantageusement un dérivé sodique d'amine et/ou au moins un groupe $NO_2$ est réduit en un groupe $NH_2$.

**10.** Procédé suivant les revendications 8 et 9, **caractérisé en ce que**, après la réaction avec BuLi, un produit du procédé portant au moins un groupe $C(O)OCH_3$ et/ou un groupe $C(S)OCH_3$ est saponifié avec une solution de KOH ou une solution de NaOH dans du méthanol à une température de 40 à 60°C.

**11.** Médicament, contenant au moins un composé de formule générale I ou l'un de ses sels pharmaceutiquement acceptables.

**12.** Utilisation d'un composé de formule générale I ou de l'un de ses sels pharmaceutiquement acceptables pour la préparation d'un médicament destiné au traitement de dépressions, d'angoisses et/ou de douleurs.

**13.** Composition pharmaceutique qui contient au moins un composé de formule générale I ou l'un de ses sels pharmaceutiquement acceptables ainsi qu'au moins une substance pharmaceutique auxiliaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **PACHER, P. ; KOHEGYI, E. ; KECSKEMETI, V. ; FURST, S.** *Current Medicinal Chemistry,* 2001, vol. 8, 89-100
- **GODDARD, A. W. ; COPLAN, J.D. ; GORMAN, J. M. ; CHARNEY, D. S.** Neurobiology of mental illness. Oxford Univerity Press, 1999, 548-563
- Anesthesia. **YAKSH, T.L. et al.** Biological foundations. Lippincott-Raven, 1997, 987-997
- **RADL et al.** *Collection of Czechoslovak Chemical Communications,* 1999, vol. 64, 377-388
- Remington's Pharmaceutical Sciences. Mack Publishing Company, 1985
- **GRAY, E.G. ; WHITTAKER, V.P.** *J. Anat.,* 1962, vol. 76, 79-88
- **FRINK, M.CH. ; HENNIES, H.-H. ; ENGLBERGER, W. et al.** *Arzneim.-Forsch./Drug Res.,* 1996, vol. 46 (III), 1029-1036
- **CHENG, Y.C. ; PRUSOFF, W.H.** *Biochem. Pharmacol.,* 1973, vol. 22, 3099-3108
- **LINEWEAVER, H. ; BURK, D.** *J. Am. Chem. Soc.,* 1934, vol. 56, 658-666
- **DUBUISSON, D. ; DENNIS, S.G.** *Pain,* 1977, vol. 4, 161-174
- **PORSOLT, R. et al.** *Arch. Int. Pharmacodyn.,* 1977, vol. 229, 327-336
- **FISH, E.W. ; SEKINDA, M. ; FERRARI, P. F. ; DIRKS, A. ; MICZEK, K. A.** *Psychopharmacology,* 2000, vol. 149, 277-285